# EUROPEAN PATENT SPECIFICATION

(11) **EP 3 714 788 B1**
(45) Date of publication and mention of the grant of the patent: **25.01.2023**
(21) Application number: 20154429.3
(22) Date of filing: 26.02.2010
(51) Int. Cl.: A61B 5/145

(54) **METHOD OF MAKING IMPROVED ANALYTE SENSORS**
VERFAHREN ZUR HERSTELLUNG VON VERBESSERTEN ANALYTSENSOREN
PROCÉDÉ DE FABRICATION DE CAPTEURS D'ANALYTE AMÉLIORÉS

(30) Priority: 26.02.2009 US 15588909 P; 26.02.2009 US 15589109 P; 26.02.2009 US 15589309 P; 31.03.2009 US 16549909 P; 31.03.2009 US 23846409 P; 29.12.2009 US 29084709 P
(43) Date of publication of application: 30.09.2020
(62) Divisional of application: 10746954.6
(73) Proprietor: Abbott Diabetes Care, Inc., Alameda, CA 94502 (US)
(72) Inventor: THOMAS, Christopher, Allen, San Leandro, CA California 94579 (US); HOSS, Udo, San Ramon, CA 94583 (US); MCGARRAUGH, Geoffrey, Oakland, CA California 94611 (US); HAYTER, Gary, Alan, Oakland, CA California 94618 (US)
(74) Representative: Booth, Catherine Louise

(56) References cited:
- WO-A1-94/20602
- US-A1- 2004 106 857
- US-A1- 2005 211 572
- US-A1- 2006 183 178
- US-A1- 2007 007 133
- US-A1- 2008 319 295

## Description

### PRIORITY

The present application claims priority to U.S. provisional application nos. 61/155,889 filed February 26, 2009 entitled "Analyte Measurement Sensors and Methods for Fabricating the Same", 61/155,891 filed February 26, 2009 entitled "Analyte Measurement Sensors and Methods for Fabricating the Same", 61/155,893 filed February 26, 2009 entitled "Analyte Measurement Sensors and Methods for Fabricating the Same", 61/165,499 filed March 31, 2009 entitled "Analyte Measurement Sensors and Methods for Fabricating the Same", 61/238,461 filed August 31, 2009 entitled "Analyte Measurement Sensors and Methods for Fabricating the Same", and 61/290,847 filed December 29, 2009 entitled "Implantable Analyte Sensors for Use with Continuous Analyte Measurement Systems and Methods for Packaging the Sensors".

### BACKGROUND

Continuous glucose monitoring (CGM) systems typically provide a comprehensive picture of monitored glucose levels of a subject. The advantages of such a system for patients diagnosed with Type 1 or Type 2 diabetes are evident. Commercially available CGM systems typically use a percutaneously or transcutaneously placed glucose sensor over a time period spanning several days to approximately a week, during which time period the real time glucose information is monitored and provided to the patient to take any necessary corrective actions for purposes of controlling potential glycemic excursions. Typical glucose sensors are manufactured in batches or lots and after each use (for the intended three, five, seven days or some other prescribed time period), are discarded and replaced with a new sensor.

Furthermore, existing CGM systems require periodic calibration of the glucose sensors which involve performing finger prick tests to determine blood glucose concentration and using the determined concentration information to periodically calibrate the sensor. Calibration is necessary to compensate for sensitivity variations between the manufactured sensors, and sensor stability drift over time, among others. The inconvenience to the patient in addition to the real and perceived pain associated with the frequent *in vitro* blood glucose testing for sensor calibrations is substantial.

Accordingly, it would be desirable to provide *in vivo* sensors for use in continuous analyte monitoring systems that do not require any sensor calibration to be performed either by the user or by the system during *in vivo* use.

US 2007/007133 A1 discloses a layered electrochemical biosensor.

US 2005/211572 A1 discloses a method and sensor for monitoring an analyte concentration.

### SUMMARY

The invention is set out in the independent claim. Preferable features are set out in the dependent claims. Improved in vivo analyte sensors, methods of making the improved sensors, and methods of using the improved sensors, are provided. The disclosure includes *in vivo* analyte monitoring devices, *e.g.,* glucose monitoring devices, methods, systems, manufacturing processes, and post manufacturing processes such as post manufacturing storage processes, that provide for analyte monitoring devices which do not require user calibration after *in vivo* positioning of the devices in the user.

Examples of devices and methods that exhibit stability profiles and/or sensitivity profiles that do not change by more than a clinically significant amount over the life of the device and/or have predictable stability profiles and/or sensitivities are provided.

The disclosure includes manufacturing process(es), for example including a calibration factor or parameter, *e.g.,* a device sensitivity, that is determined (empirically, statistically or theoretically, for example) during the manufacturing for one or a plurality of analyte sensor lots, and assigned to the one or more lots, *e.g.,* recorded in memory or suitable storage device for the manufactured one or more sensor lots (and/or coded on the sensors in the lots themselves). The calibration factor may be used by the devices when the sensors are positioned in the body of users for active analyte monitoring to conform the sensors to a standardized value, including conform analyte data obtained therefrom (*e.g*., current signals obtained from the sensor and measured in Amperes) from interstitial fluid to blood glucose data (*e.g.,* in units of mg/dL). For example, the disclosure includes sensors from the same and/or different lots that are assigned and use the same calibration factor, and the calibration factor is determined prior to the manufacture of the given sensor lot(s), e.g., using historical data from prior lot(s).

The disclosure includes sensor lots and the sensors therefrom in which sensors from the same and/or different manufacturing lots are assigned and use the same calibration factor, and the calibration factor is determined substantially contemporaneously to the manufacture of one or more including all, of the given lots, e.g., in real time relative to manufacture.

The disclosure includes sensor manufacturing lots with extremely low sensor sensitivity coefficient of variation (CV) within and/or between sensor lots. For example, CVs as low as about 5% or lower, *e.g.,* as low as about 3% or lower, *e.g.,* as low as about 2% or lower, *e.g.,* as low as about 1% or lower. In certain embodiments, the extremely low CVs are achieved at least by one or more robust manufacturing processes.

No user calibration analyte monitoring devices and methods also include embodiments that have extremely high sensor stability in a given user over the life of the sensor. For example, the sensor stability profile in a user may not change by more than a clinically and/or statistically significant amount over the sensor lifetime. For example stability may not change by more than about 5% or lower, *e.g.,* as low as about 3% or lower, *e.g.,* as low as about 2% or lower, *e.g.,* as low as about 1% or lower.

As discussed above, the disclosure includes *in vivo* analyte monitoring devices having extremely low variability/high precision in the thickness of the flux limiting membrane within a manufacturing lot and/or between manufacturing lots. Manufacturing techniques and processes provide reproducible active sensing area of the sensor working electrode with controlled and substantially uniform membrane thickness such that a coefficient of variation (CV) of about 5% or less, *e.g.,* 3% or less, e.g., about 2% or less e.g., about 1 % or less, in sensor to sensor sensitivity amongst the manufactured lot or batch of sensors is obtained.

The disclosure includes *in vivo* manufacturing techniques and processes, for example that provide controlling the area of the sensor working electrode and/or the membrane thickness, *e.g.,* to control the sensor sensitivity across manufactured lots or batches. As the glucose concentration on working electrode surface (for example, the active sensing area) is proportional to the thickness of the membrane and the sensitivity is proportional to the area of the working electrode, by selective and precise control of the membrane thickness and the area (*e.g*., active area) of the working electrode of the sensor, sensors may be manufactured that do not require any calibration by the user nor by the CGM system.

In addition, in further aspects of the present disclosure, the glucose limiting membrane of the analyte sensors provides biocompatibility when positioned or placed *in vivo* such that any potential biofouling or suspected biofouling is minimal and does not adversely contribute to the *in vivo* stability of the sensor so as to require *in vivo* calibration. For example, the analyte sensor exhibits in one embodiment, about 2% to about 3% change or less, for example, about 1% to about 2% change or less, or in a further aspect, less than about 1% change in *in vivo* sensor sensitivity stability over the sensor sensing time period (*e.g.,* three days, five days, seven days, 14 days, or more), which would not require user or system based calibration during *in vivo* use.

The disclosure includes reproducible active areas of analyte sensors, where the sensing chemistry is provided on the working electrode of the sensor. The working area may have dimensional ranges of about 0.01mm² to about 1.5 mm² or less, for example, about 0.0025 mm² to about 1.0 mm² or less, or for example, about 0.05 mm² to about 0. 1 mm² or less. Embodiments also include reproducible active areas of the sensors with voids or wells. Active area voids/wells may have dimensional ranges of about 0.01mm² to about 1 .0 mm³ or, for example, about 0.04 mm² to about 0.36 mm². The dimensions of the void/well at least partially define the shape (and thus the size) of the active area of the sensor. The shape of the void/well may be varied to achieve the same desired volume and/or surface area. For example, the height of the void/well may be gradually increased or decreased. In addition, the surface area of the void/well may be shaped such that it is tapered, or otherwise varied, including, for example, a triangular shape, an oblong shape, and the like.

The disclosure further includes reproducible sensor constructs including precise dimensions of the sensor distal portion. The width of a conductive layer of the sensor may be governed by the substrate width of the sensor distal portion. The active area of the sensor may range from about 0.0025 mm² to about 3 mm², for example, from about 0.01 mm² to about 0.9 mm².

The disclosure further includes analyte sensors having sensing and conductive layers, *e.g.,* in the form of stripes or the like, with substantially constant widths and provided orthogonal to each other (for example, an orthogonal relationship between the sensing layer and the conductive layer) to form a substantially constant active area along the length and the width of the sensor distal portion.

Moreover, the disclosure also includes precise laser processes, e.g, laser ablation techniques, to remove, trim, modify or ablate excess or undesired material from the sensor body and precisely define and reproduce the desired active area of the sensors which have clinically insignificant CV and that do not require user initiated or CGM system based calibration to report accurate real time monitored glucose levels during the useful life of the *in vivo* sensors.

The disclosure further includes post manufacturing and pre *in vivo* use storage techniques including sensor packaging with controlled and/or minimal adverse environmental effects upon the packaged sensors prior to *in vivo* use, *e.g.,* to minimize sensor stability degradation during storage. For example, embodiments include sensor packaging techniques that maintain the moisture and vapor transmission rate (MVTR) to about 0.5 mg/day or less, for example, about 0.46 mg/day or less, for example, about 0.4 mg/day or less. Desiccant material may be provided on, in, within or with the sensor packaging to maintain a substantially stable environment during the sensor's shelf life (*e.g*. , about 0 to about 24 months, *e.g.,* 0 to about 18 months).

The disclosure includes *in vivo* glucose sensors that provide predictable and stable *in vivo* sensor sensitivity, and methods for compensating for inter and intra subject variation in *in vivo* response is provided, obviating the need or requirement to perform sensor calibration during *in vivo* use, *i.e*., no calibration by the user and/or the CGM system during this time period.

The disclosure further includes *in vivo* sensors that do not require factory calibration, and further, that do not require user or system executed or implemented sensor calibration. That is, in certain aspects, the manufactured *in vivo* sensors exhibit characteristics that include substantially stable sensitivity profiles post manufacturing and during *in vivo* use. For example, interferents such as oxygen, acetaminophen or ascorbic acid that may be present during in *vivo* sensor use may be m inimized by careful selection of the sensor membrane material (*e.g*., membrane that has low oxygen permeability), the sensing chemistry (*e.g*., designed or selected to have minimal effects by the interferents such as oxygen) in addition to well defined and reproducible active areas of the sensor, sensor geometry, and tightly controlled post manufacturing environment during sensor shelf life.

Feedback algorithms may be programmed or programmable in the CGM system to provide or compensate for variation in the interstitial to blood glucose concentration between each *in vivo* environment (*e.g*., between each subject using the *in vivo* sensors) such that sensor sensitivity is compensated or corrected during *in vivo* use based on, for example, a stability profile determined *a priori* for each subject and applied to the signals received from the *in vivo* sensors during use. Such algorithms or routines may be generated or determined based on prior *in vivo* sensor feedback signals and programmed or programmable (and subsequently modifiable) in the CGM system and applied to the signals received from the sensor during *in vivo* use.

In the manner described, the present disclosure provides *in vivo* sensors and CGM systems employing *in vivo* sensors and manufacturing and packaging the same that, for example, but not limited to, require no user initiated or based calibration, that do not require system based calibration, that do not require factory based calibration, that only require a system executed calibration (for example, automatically executed or implemented one or more calibration routines), or that require only a single user initiated calibration or sensitivity confirmation over the sensor life during *in vivo* use of the sensor.

### BRIEF DESCRIPTION OF THE DRAWINGS

A detailed description of various aspects, features and embodiments of the present disclosure is provided herein with reference to the accompanying drawings, which are briefly described below. The drawings are illustrative and are not necessarily drawn to scale, with some components and features being exaggerated for clarity. The drawings illustrate various aspects or features of the present disclosure and may illustrate one or more embodiment(s) or example(s) of the present disclosure in whole or in part. A reference numeral, letter, and/or symbol that is used in one drawing to refer to a particular element or feature maybe used in another drawing to refer to a like element or feature. Included in the drawings are the following:
FIG. 1 illustrates a planar view of an analyte sensor in accordance with one aspect of the present disclosure;
FIG. 2 illustrates a planar view of an analyte sensor in accordance with another aspect of the present disclosure;
FIG. 3A illustrates a top planar view of the tail or distal end of the analyte sensor of FIG. 1 for fluid contact with an interstitial fluid during *in vivo* use in one aspect;
FIG. 3B illustrates a side cross sectional view at line B of the analyte sensor at the distal end as shown in FIG. 3 in one aspect;
FIGS. 4A and 4B illustrate an analyte sensor configuration in accordance with another embodiment of the present disclosure;
FIGS. 5A and 5B illustrate a top planar view and a cross sectional view, respectively, of an analyte sensor in one aspect;
FIGS. 6A and 6B illustrate a top planar view and a cross sectional view, respectively, of an analyte sensor in another aspect;
FIGS. 7A and 7B illustrate a top planar view and a cross sectional view, respectively, of an analyte sensor in yet another aspect;
FIGS. 8A and 8B illustrate a top planar view and a cross sectional view, respectively, of an analyte sensor in yet still another aspect;
FIGS. 9A-9C illustrate top, bottom and cross sectional side views, respectively, of a two sided analyte sensor in accordance with one aspect;
|0034] FIGS. 10A-10C illustrate top, bottom and cross sectional side views, respectively, of a two sided analyte sensor in accordance with one aspect;
FIGS. 11A-11C illustrate top and cross-sectional side and end views, respectively, of an analyte sensor prior to laser trimming of the sensor's sensing layer in accordance with one aspect;
FIGS. 12A-12C illustrate top and cross-sectional side and end views, respectively, of the analyte sensor of FIGS. 11A-11C after laser trimming of the sensor's sensing layer in accordance with one aspect;
FIGS. 13A-13C illustrate top and cross-sectional side and end views, respectively, of an analyte sensor prior to laser trimming of the sensor's sensing and working electrode layers in accordance with another aspect;
FIGS. 14A-14C illustrate top and cross-sectional side and end views, respectively, of the analyte sensor of FIGS. 13A-13C after laser trimming of the sensor's sensing and working electrode layers in accordance with another aspect;
FIGS. 15A-15C illustrate top and cross-sectional side and end views, respectively, of an analyte sensor prior to laser trimming of the sensor's sensing and working electrode layers in still another aspect;
FIGS. 16A-16C illustrate top and cross-sectional side and end views, respectively, of the analyte sensor of FIGS. 15A-15C after laser trimming of the sensor's sensing and working electrode layers in accordance with still another aspect;
FIG. 17 shows an exploded perspective view of one embodiment of a packaged sensor assembly of one aspect of the present disclosure;
FIG. 18 shows an assembled perspective view of one embodiment of the packaged sensor assembly of FIG. 17;
FIGS. 19A-19C show side, bottom and end views, respectively, of the tray component of the packaging of FIG. 17;
FIG. 20A illustrates a top view of a working electrode of an analyte sensor in one embodiment of the present disclosure;
FIG. 20B illustrates a cross-sectional view at line B of FIG. 20A;
FIG. 20C illustrates a cross-sectional view at line C of FIG. 20A;
FIGS 21A-21D illustrate stages of sensing layer application to the working electrode shown in FIG. 217A in one embodiment of the present disclosure;
FIG. 22 illustrates an exemplary time varying sensitivity drift profile associated with an analyte sensor in accordance with one embodiment of the present disclosure;
FIG. 23 illustrates sensitivity variation of 16 analyte sensors from a sensor lot manufactured in accordance with one of more processes) of the present disclosure in response to a beaker solution with known glucose concentration;
FIG. 24 illustrates response of the sensors from the same lot as described in conjunction with FIG. 23 in one aspect; and
FIG. 25 is a Clarke Error Grid based on analyte sensors manufactured in accordance with the one or more embodiments of the present disclosure.

### RELATED APPLICATIONS

The following patents, applications and/or publications describe related analyte sensors and systems: U.S. Patent Nos. 4,545,382; 4,711,245; 5,262,035; 5,262,305; 5,264,104; 5,320,715; 5,509,410; 5,543,326; 5,593,852; 5,601,435; 5,628,890; 5,820,551; 5,822,715; 5,899,855; 5,918,603; 6,071,391; 6,103,033; 6,120,676; 6,121,009; 6,134,461; 6,143,164; 6,144,837; 6,161,095; 6,175,752; 6,270,455; 6,284,478; 6,299,757; 6,338,790; 6,377,894; 6,461,496; 6,503,381; 6,514,460; 6,514,718; 6,540,891; 6,560,471; 6,579,690; 6,591,125; 6,592,745; 6,600,997; 6,605,200; 6,605,201; 6,616,819; 6,618,934; 6,650,471; 6,654,625; 6,676,816; 6,676,819; 6,730,200; 6,736,957; 6,746,582; . 6,749,740; 6,764,581; 6,773,671; 6,881,551; 6,893,545; 6,932,892; 6,932,894; 6,942,518; 7,167,818; and 7,299,082; U.S. Published Application Nos. 2004/0186365; 200510182306; 2007/0056858; 2007/0068807; 2007/0227911; 2007/0233013; 2008/0081977; 2008/0161666; and 2009/0054748; U.S. Patent Application Serial Nos. 12/131,012; 12/242,823; 12/363,712; 12,698,124; and 12/981,129; and U.S. Provisional Application Serial Nos. 61/149,639; 61/155,889; 61/155,891; 61/155,893; 61/165,499; 61/230,686; 61/227,967 and 61/238,461.

### DETAILED DESCRIPTION

Before the present disclosure is described, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges is also encompassed within the disclosure, subject to any specifically excluded limit in the stated range. Where the stated range includes one or both of the limits, ranges excluding either or both of those included limits are also included in the disclosure.

It must be noted that as used herein and in the appended claims, the singular forms "a", "an", and "tire" include plural referents unless the context clearly dictates otherwise.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure.

Embodiments of the present disclosure relate to methods and devices for detecting at least one analyte, such as glucose, in body fluid. Embodiments relate to the continuous and/or automatic *in vivo* monitoring of the level of one or more analytes using an analyte monitoring system that includes an analyte sensor for the *in vivo* detection of an analyte, such as glucose, ketones, lactate, and the like, in a body fluid. Embodiments include wholly implantable analyte sensors and transcutaneous analyte sensors in which only a portion of the sensor is positioned under the skin and a portion of the sensor resides above the skin, *e.g.,* for contact to a control unit, transmitter, receiver, transceiver, processor, etc. At least a portion of a sensor may be constructed for subcutaneous positioning in a patient for monitoring of a level of an analyte in a patient's interstitial fluid over a time period such as for example, about three days or more, about five days or more, about seven days or more, about ten days or more, about fourteen days or more, *e.g.,* or based on the sensor life determined, for example, by the sensor characteristics such as the sensing chemistry formulation of the sensor to provide accurate sensing results, and/or the sensor packaging and/or storage conditions or combinations thereof. For the purposes of this description, semi-continuous monitoring and continuous monitoring will be used interchangeably, unless noted otherwise.

Embodiments include analyte sensors. A sensor response may be obtained and correlated and/or converted to analyte levels in blood or other fluids. In certain embodiments, an analyte sensor may be positioned in contact with interstitial fluid to detect the level of glucose, which detected glucose may be used to infer the glucose level in the patient's bloodstream. Analyte sensors may be insertable into a vein, artery, or other portion of the body containing fluid. Embodiments of the analyte sensors of the subject disclosure are configured to substantially continuously monitor the level of the analyte over a sensing or monitoring time period which may range from minutes, hours, days, weeks, months, or longer, and to generate analyte related signals (for example, in pre or post processed signals to be converted in to the corresponding glucose measurement values during the sensing time period).

Analytes that may be monitored include, but are not limited to, acetyl choline, amylase, bilirubin, cholesterol, chorionic gonadotropin, creatine kinase (*e.g.,* CK-MB), creatine, creatinine, DNA, fructosamine, glucose, glutamine, growth hormones, hormones, ketone bodies, lactate, oxygen, peroxide, prostate-specific antigen, prothrombin, RNA, thyroid stimulating hormone, and troponin. The concentration of drugs, such as, for example, antibiotics (*e.g*., gentamicin, vancomycin, and the like), digitoxin, digoxin, drugs of abuse, theophylline, and warfarin, may also be monitored. In those embodiments that monitor more than one analyte, the analytes may be monitored at the same or different times.

Embodiments of the sensor or sensor systems include *in vivo* analyte sensors for use in analyte monitoring systems such as a continuous glucose monitoring systems which does not require calibration during *in* vivo use. More specifically, factory calibration systems in certain aspects include *in vivo* analyte monitoring systems with analyte sensors that do not require any reference analyte tests, *e.g., in vitro* finger stick glucose tests or YSI tests, or the like, and related calibration of the *in vivo* sensor data using the results of those reference tests by the user during *in vivo* use. Advantages of the systems herein, including factory calibrated systems and/or no user calibrated systems are evident including reducing the inconvenience to the user by eliminating the need for the periodic *in vitro* finger test glucose tests, and reducing the potential source of error in the *in vivo* sensor readings during use.

Embodiments further include *in vivo* sensors that provide characteristics which include sensor to sensor reproducibility within each manufactured sensor lot and/or between sensor lots. An exemplary sensor lot as referred to herein includes a batch of *in vivo* sensors that are manufactured using the same manufacturing equipment during the manufacturing process with the same material and process. Embodiments herein include sensor lot(s) manufactured with very similar or identical sensor characteristics including sensor stability profile (for example, similar or the same sensor sensitivity, shelf life characteristics, and the like). For example, a sensor lot may include 2 or more sensors, *e.g*., about 1,000 or more, about 5,000 or more, or about 10,000 or more (or other suitable manufacturable number of sensors in a lot or batch) *in vivo* sensors which are streamlined for manufacturing with the same manufacturing equipment and processes in addition to being fabricated from the same materials including substrate or non conductive material, conductive material for the electrodes, sensing chemistry composition, the sensor membrane characteristics such as thickness, size and other physical and/or chemical properties. The sensor lot defined herein is for exemplary purposes only and the number of sensors manufactured as a lot is constrained largely by the capacity supported by the equipment for manufacturing the same. To this end, in accordance with embodiments of the present disclosure, the sensor lot may be greater or fewer than the approximately 1,000 *in vivo* sensors of exemplary embodiments herein.

Embodiments of the *in vivo* sensors have post manufacturing shelf life stability such that degradation in sensor sensitivity prior to *in vivo* use is minimized, including eliminated, and any variation in shelf life stability is minimal or insignificant or is null. Embodiments include packaging of sensor and/or sensor systems that employ desiccants and/or other materials to provide a stable shelf life environment to maintain, for example, the effectiveness of the sensors and/or sensor systems during storage and prior to *in* vivo use.

Embodiments of the sensors may be used in analyte monitoring systems that implement data processing techniques and/or signal compensation to adjust or compensate for the variation in sensor response during *in vivo* use to minimize intra and inter subject variability of sensor sensitivity. Such embodiments may include compensation for early signal attenuation of the sensor signals during the initial implantation time period and during which spurious or transient signals from the sensors are detected.

Embodiments further include calibration code or parameter which may be derived or determined during one or more sensor manufacturing processes and coded or programmed, as part of the manufacturing process, in the data processing device of the analyte monitoring system or on the sensor itself, for example, as a bar code, a laser tag, an RFID tag, or other machine readable information provided on the sensor, or a physical configuration of the sensor from which the calibration code or parameter information may be derived (for example, such as based on a size including for example one or more of a height, a width, a circumference, a diameter, a surface area, a volume or one or more combinations thereof, of a formation or an indentation on a surface of the sensor body, a position of a formation or an indentation on the surface of the sensor body,) such that user initiated sensor calibration during *in vivo* use of the sensor is obviated, or the frequency of *in vivo* calibrations during sensor wear is reduced. In embodiments where the calibration code or parameter is provided on the sensor itself, prior to or at the start of the sensor use, the calibration code or parameter may be automatically transmitted or provided to the data processing device in the analyte monitoring system.

A plurality of analyte systems from the same and/or different lots may include the same calibration code, including all of the analyte systems manufactured by a given manufacturer over a period of time such as over about 1 day to about 1 year or more, *e.g.,* more than 1 year.

Embodiments include sensors and sensor systems where the calibration code or parameter determined during sensor manufacturing may be sensor specific or lot specific, and upon determination, provided to the data processing device of the analyte monitoring system automatically, or manually. For example, the determined calibration code or parameter for a particular manufactured sensor may be provided in the sensor packaging such that, prior to *in vivo* use, the user may be required to manually input the code or parameter into the data processing device in the analyte monitoring system.

As discussed in further detail below, embodiments of the analyte sensors of the present disclosure include sensors manufactured with techniques and procedures to control the active area(s) of the sensors, including a glucose sensing layer on the working electrode and/or a glucose limiting membrane. For example, analyte sensors in accordance with embodiments of the present disclosure provide (1) reproducible active area of the sensor, (2) uniform sensor membrane thickness and composition, (3) stable active enzymes, and (4) predictable biocompatibility. For example, as the flux of the glucose to the working electrode is proportional to the thickness of the sensor membrane, sensors manufactured with a substantially uniform membrane thickness provide sensors that do not require *in vivo* calibration by the user, *i.e.,* they may be factory calibrated or require no calibration post manufacturing and during *in vivo* use.

### Overall Sensor Structure

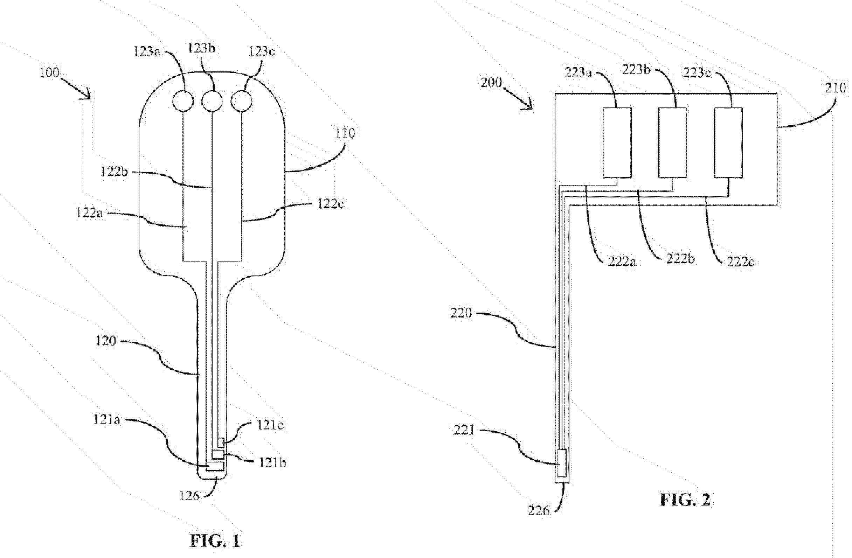

FIG. 1 illustrates a planar view of an analyte sensor in accordance with one aspect of the present disclosure. Referring to FIG. 1, in one embodiment, analyte sensor 100 includes sensor body having a proximal section 110 and a distal section 120. The distal end 126 of the distal section 120 of the sensor 100 may have a width appropriate or suitable for transcutaneous positioning through a skin surface of a user. For example, in one aspect, the distal section 120 may be dimensioned to have a width of about 2 mm or less, or about 1 mm or less, or about 0.5 mm or less, or about 0.3 mm or less, or about 0.25 mm or less to define a distal tip 126 for insertion under the skin layer of the user.

In certain aspects, as illustrated in FIG. 1, conductive material is disposed on the sensor 100. The conductive material may include one or more electrodes 121a, 121b, 121c, conductive traces 122a, 122b, 122c and contacts 123a, 123b, 123c. In one embodiment, one or more electrodes 121a, 121b, 121c are disposed near the distal end 126 of distal section 120 of the sensor 100. In this manner, the one or more electrodes 121a, 121b, 121c are implanted in the tissue of a user in fluid contact with an interstitial fluid, for example, to detect and measure the analyte of interest in the bodily fluid. The signals generated by the analyte sensor are communicated via the conductive traces 122a, 122b, 122c and eventually to transmitting circuitry, described below. The one or more electrodes 121a, 121b, 121c may include one or more working electrodes, one or more counter electrodes, one or more reference electrodes, or one or more combinations thereof. In one embodiment, the sensor 100 may include three electrodes, *e.g.,* a working electrode, a counter electrode, and a reference electrode. Other embodiments, however, can include less or more electrodes, such as described in U.S. Patent Application Nos. 61/247,519, and 12/393,921. Yet in still further embodiments, multiple working electrodes may be provided on the sensor. The electrodes 121a, 121b, 121c of FIG. 1 are illustrated in a side by side configuration, however, other electrode configurations may be used, including, but not limited to, a stacked configuration. Further, embodiments of the sensor in accordance with the present disclosure includes but not limited to a planar sensor, a wire sensor, a sensor having stacked or layered electrodes (for example, where the electrodes are separated by insulation or substrate materials) as well as sensors having electrodes that are co-planar and disposed side-by-side on the substrate.

Suitable conductive materials include, but are not limited to, lamp black carbon in a polymer thick film binder, vitreous carbon, graphite, silver, silver-chloride, platinum, palladium, iridium, platinum-iridium, titanium, gold, or the like. The conductive material can be applied to the sensor by various techniques including sputtering, evaporation, printing, or extrusion, or the substrate may be patterned using laser ablation, or photolithography. In certain aspects, *e.g.,* using gold as the conductive material applied to the sensor, the thickness of the gold material may be in the range of approximately 40 nm to 120 nm, *e.g.,* approximately 50 nm to 80 nm, *e.g.,* approximately 60 nm. While exemplary ranges for dimensions of the material are described above, embodiments of the present disclosure contemplates other dimensions which may be greater or less than those specified, and the scope of the present disclosure are not to be construed as being limited to the exemplary dimensions provided above.

FIG. 2 illustrates a planar view of an analyte sensor in accordance with another aspect of the present disclosure. FIG. 2 illustrates an alternative sensor configuration of the sensor 100 of FIG. 1. In one embodiment, the analyte sensor 200 illustrated in FIG. 2 includes a proximal portion 210 and a distal portion 220 including a distal tip 226. The dimensions of the distal portion 220 and distal tip 226 of the sensor 200, in one aspect, are configured to facilitate transcutaneous positioning through a skin surface of a user, as described in further detail above in conjunction with FIG. 1.

In certain aspects, the sensor 200 of FIG. 2 also includes conductive material (described in further detail above in conjunction with FIG. 1) disposed on the sensor 200 to form one or more of electrodes 221, conductive traces 222a, 222b, 222c and contacts 223a, 223b, 223c. The electrodes 221 of FIG. 2 are shown in a stacked configuration, whereby the conductive material of each electrode is stacked on one another and separated by a non-conducting dielectric layer, however, as discussed above, other configurations including, but not limited to, a side by side configuration may also be used. In other embodiments, electrodes, conductive traces, and/or contacts are provided on both sides of the sensor body. Other sensor designs and electrode configurations are also provided within the scope of the present disclosure including, but not limited to, planar and wire sensors and stacked, side by side, and twisted electrode configurations. Other exemplary sensors and electrode configurations can be found in, among others, U.S. Patent Nos. 6,175,752, 6,134,461 and 6,284,478, and US Publication No. 2007/0135697.

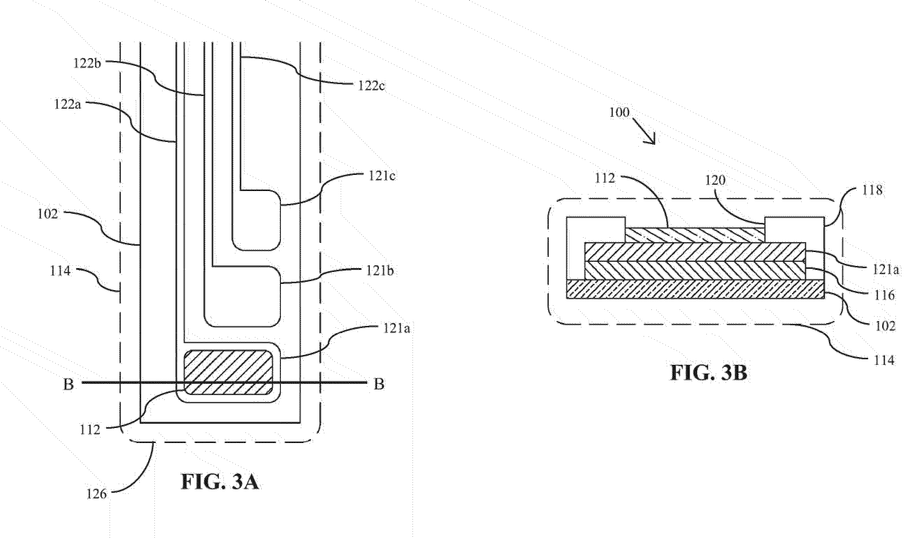

FIG. 3A illustrates a distal tip portion 126 of the distal section 120 of the analyte sensor 100 of FIG. 1 in one embodiment. In one aspect, the distal tip portion 126 of the sensor 100 is adapted for at least partial subcutaneous and/or transcutaneous positioning in the tissue of a user and in contact with bodily fluid such as the interstitial fluid. The sensor 100 in one aspect may include a substrate 102, manufactured from a polymer material, such as for example, polyester based material or polyimide.

Referring again to FIG. 3A, in one aspect, the analyte sensor 100 includes working electrode 121a, counter electrode 121b, and reference electrode 121c. Conductive traces 122a, 122b, 122c provide electrical connection between the electrodes 121a, 121b, 121c with the respective corresponding contacts 123a, 123b, 123c (FIG. 1). The sensing layer 112 used for detecting the analyte, *e.g.,* an enzyme and an optional electron transfer agent, described in detail below, are applied at least to the working electrode 121a. Sensing material (*e.g*., absent one or more components applied to the working electrode, *e.g*., absent enzyme and/or optional electron transfer agent) may be applied to one or more other electrodes. At least the distal tip portion 126 of the sensor 100 may be covered with a biocompatible membrane 114.

FIG. 3B illustrates a cross-sectional view of the distal tip portion 126 of sensor 100 in one aspect. As shown, in one embodiment, the sensor 100 includes a dielectric or substrate 102, and an optional first layer 116 that may be a conductive layer such as vitreous carbon, graphite, silver, silver-chloride, platinum, palladium, platinum-iridium, titanium, gold or, iridium , applied to the substrate 102. Layer 1 16 may be an adhesion layer using sputtering or evaporation processes. The working electrode 121a, which includes a conductive material such as vitreous carbon, graphite, silver, silver-chloride, platinum, palladium, platinum-iridium, titanium, gold or, iridium, or the like, in some embodiments, may be applied to the substrate 102 over the adhesion layer 116. In other embodiments, a conductive material may be applied only on an area on the adhesion layer 116 to form the working electrode 121a, or can be applied over an area greater than the area of the working electrode 12la on the adhesion layer 1 16, or may be applied over the entire adhesion layer 116. The edges of the working electrode 121a may be precisely defined by a procedure such as laser ablation for modifying the edges, *e.g.,* removing excess material or otherwise shaping material. A similar technique of applying the conductive material and laser ablation may also be used in connection with forming or providing the traces 122a, 122b, 122c, the counter electrode 121b, the reference electrode 12 1c, or any other area where conductive material is applied to the sensor.

In certain embodiments, the reference electrode 121c may be coated with silver/silver chloride, *e.g.,* using screen printing, extrusion or electrolytic deposition or electroplating, or the like. In certain aspects, the thickness of the conductive material such as gold applied to the sensor may be in the range of approximately 40 nm to 120 nm, *e.g.,* approximately 50 nm to 80 nm, *e.g*., approximately 60 nm. Furthermore, in one aspect, the first layer 116 may be approximately in the range of 10 nm to 30 nm, *e.g*., approximately 20 nm.

Referring again to FIG. 3B, in one aspect, a coverlay material 118 may be applied over the distal tip portion 126 of the sensor 100. In one embodiment, the coverlay material 118 is applied only over the electrodes 121a, 121b, 121c. In still other embodiments, the coverlay material is applied over the working electrode 121a, or substantially over the substantially the entire substrate 102. The coverlay material 118 may be used to encapsulate some or all of the electrodes, and provides environmental and electrical insulation. In certain aspect, the coverlay material 118 may include, for example, but not limited to, a photo imageable material, such as, polyimide or polyester based material, for example. That is, in certain embodiments, the polymers or coverlay material 118 are may be photo-imageable to allow portions of the polymers to be removed. *e.g*., for exposure of contacts and/or sensor electrodes for application of sensor chemistry, or the like. In certain aspects of the present disclosure, portions of the coating polymer or the coverlay material 118 may be masked to form a pattern, which is then exposed and developed to remove the portions of the polymer coating for further processing of the sensor. In certain aspects, the coating polymer may be removed by other methods, such as by laser ablation, chemical milling, or the like. Also, a secondary photo resist may be used to target specific areas of the polymer or coverlay material 118 for removal during the sensor manufacturing process.

In certain aspects, an opening 120 such as a void or a well may be created or defined in the coverlay material 118, *e.g.,* using photolithographic techniques, such as photo-etching to a depth sufficient to expose one or more of the electrodes, *e.g*., the working electrode 121a. The photolithography technique in certain embodiments uses positive or negative photoresists where the exposed portion becomes soluble or insoluble after exposure, respectively. The solubilized portions are the removed via a washout or develop, etch and strip step following exposure. The exposure process in certain aspects uses a precision mask aligner that aligns a photomask (for example, over the coverlay material 118) via an (X, Y) or (X, Y, theta) stage to the existing metal layer using fiducial features in the metal layer specific to the mask aligner vision mechanism before the mask exposure step occurs. The mask exposure step exposes the desired section(s) of the photomask to, for example, UV light that changes the solubility of the exposed portion of the photomask. The photomasks in certain embodiments are made of material that is transparent at the UV wavelengths such as quartz, glass or polyester.

The sensing layer 112 used for reacting with the analyte is then disposed in the formed void or well shown as the opening 120 over the working electrode 121a. In certain embodiments, one or more sensing layer components may be deposited on one or more other electrodes. As further shown in FIG. 3B, the biocompatible membrane 114 surrounds the distal tip portion 126 of the sensor 100. In other embodiments, the biocompatible membrane 114 can surround the entire portion of the sensor 100 configured for transcutaneous positioning.

In certain aspects of the present disclosure, the coverlay material 118 that is disposed over the one or more of the electrodes to partially or fully coat the one or more electrodes may include, for example, a non-conductive polymer. Suitable insulating materials include but are not limited to polyethyleneterephthalate, parylene, fluorinated polymers, polyurethane, polyimide, other non-conducting polymers, glass or ceramics. The insulating material may be coated on the electrodes by various coating methods, including but not limited to chemical or physical vapor deposition, hot roller lamination, spray coating, dip coating, slot-die extrusion, direct coating, or other coating techniques. In some embodiments, the insulating coating is partially or selectively stripped away from the electrode to expose an electroactive surface. In some embodiments, an insulating substrate (*e.g.,* dielectric material) and electrodes can be arranged in a stacked orientation (*i.e.,* insulating substrate disposed between electrodes). In another embodiment, the electrodes may be arranged in a side by side orientation, as described in U.S. 6,175,752.

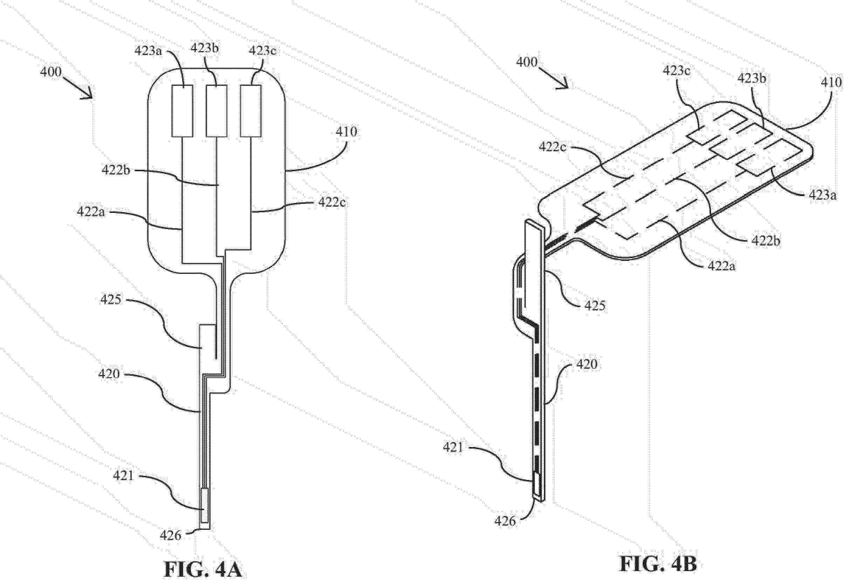

FIGS. 4A and 4B illustrate an analyte sensor configuration in accordance with another embodiment of the present disclosure. More specifically, FIG. 4A illustrates the planar view of the sensor substrate body and FIG. 4B illustrates the sensor body configured with a bend or angulation for transcutaneous placement through the skin layer and in fluid contact with the interstitial fluid. As can be further seen from FIG. 4A, the layout/configuration of the various electrodes, conductive traces and contacts, as compared to the embodiments shown in FIGS. 1 and 2 can be different. However, the distal tip portion of the sensor shown in FIG. 4A that is configured for subcutaneous and/or transcutaneous placement may be similar or the same in construct and/or in layout as that shown in FIGS. 3A and 3B.

Referring to FIGS. 4A and 4B, in one embodiment, the sensor 400 includes a proximal portion 410, a distal portion 420, and an intermediate portion 425. The intermediate section 425 maybe provided at a predetermined angle relative to position and/or orientation from the distal section 420. For example, intermediate section 425 may be laterally displaced or staggered from distal section 420. To this end, a gap may be defined between the intermediate section 425 and the distal section 420. The gap may have a consistent spacing along its length such that the primary axes of the intermediate 425 and distal 420 sections remain parallel to each other, or may have a variable spacing along its length.

Still yet, as shown in FIG. 4B, the proximal section 410 of the sensor 400 may be provided at a predetermined position and/or orientation relative to the intermediate section 425 and/or the distal section 420. In this manner, a second gap may be defined between the proximal section 410 and the intermediate section 425 of the sensor body 400 where at least a portion of proximal section 410 is laterally displaced from intermediate section 425. Intermediate section 425 and the corresponding gaps between the intermediate section 425 and the proximal 410 and distal 420 sections may be configured such that intermediate section 425 is used to assist with removal of an insertion sharp (for example, a introducer needle) used during sensor insertion and subsequent removal or withdrawal of the introducer needle or the insertion sharp from the user or the patient after sensor insertion or positioning under the skin layer.

Still referring to FIGS. 4A and 4B, in certain aspect, the sensor 400 of FIGS. 4A and 4B also includes conductive material (described in further detail above in conjunction with FIG. 1) disposed on the sensor 400 to form one or more of electrodes 421 at a distal tip portion 426 configured to facilitate transcutaneous positioning through a skin surface of a user, conductive traces 422a, 422b, 422c and contacts 423a, 423b, 423c. In one embodiment, the conductive material is not disposed on the intermediate portion 425 of the sensor 400. Various other configuration and/or layouts of the electrodes 421, conductive traces 422a, 422b, 422c and contacts 423a, 423b, 423c, such as, but not limited to, the layouts and configurations associated with FIGS. 1 and 2, are also included within the scope of the present disclosure, including, for example, co-planar or co-axial positioning or orientation of the conductive traces 422a, 422b, 422c and contacts 423a, 423b, 423c and the corresponding electrodes of the sensor, staggered or stacked or layered electrodes of the sensor, or two sided sensor configuration including electrodes provided on both sides or surfaces of the substrate.

### Active Area of Sensor

In certain embodiments, *in vivo* sensors in accordance with the present disclosure have reproducible active areas of the working electrode. That is, for each manufactured sensor, the parameters or characteristics of the active area (defined as the area of the sensing chemistry on the working electrode) are reproducible such that the coefficient of variation (CV) of the active area is less than about 5% between sensors within the sensor lot, for example, less than about 3%, for example, less than about 1%. This may be achieved by manufacturing process control and defined procedures during the *in vivo* sensor manufacturing where the active area is accurately defined.

The reproducibility of the active area of the sensor in one aspect minimizes the variation sensitivity between sensors by maintaining substantially constant the dimensions (width, length, diameter and thickness) of the active area, *i.e.,* the area of the working electrode in contact with the sensing component among the manufactured sensors.

In certain embodiments, the active area of the working electrode may be undefined until such time during the manufacturing process when the values (for example, related to viscosity or permeability of the membrane polymer lot used, or the activity of the enzyme used for the lot) magnitude or range or variation of such values related to parameters that affect manufacturing precision (thus effecting reproducibility), for example, on a sensor lot by sensor lot basis are determined, understood, analyzed or otherwise acquired. For example, the area of the working electrode and the enzyme/sensing layer spot may be left larger than the final desired active area of the working electrode until the values related to the parameters discussed above are determined, understood, analyzed or otherwise acquired, at which time, the active area of the working electrode may be trimmed to the desired size or geometry. The trimming process may be one of the laser based processes described below, including for example, ultraviolet (UV), infrared (IR) laser, or short pulse delivered or provided via a scanner, fixed beam, or ablation mask, for example.

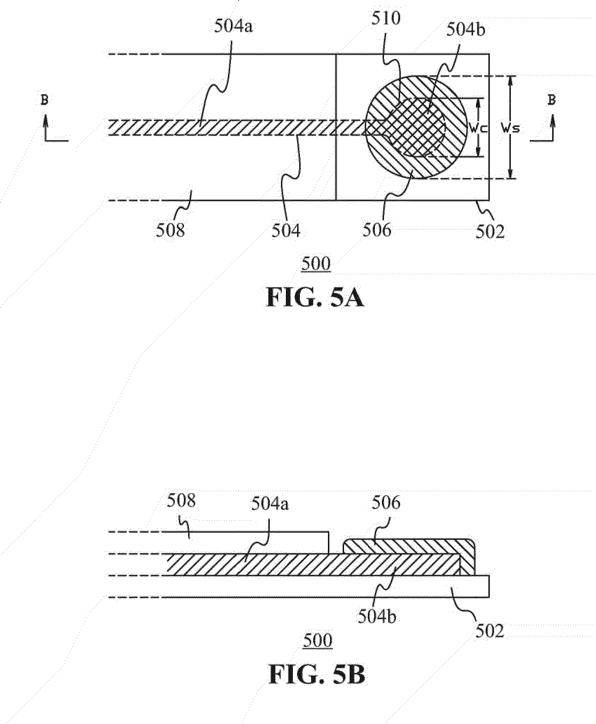

FIGS. 5A and 5B illustrate a top planar view and a cross sectional view respectively, of an analyte sensor in one aspect of the present disclosure. More specifically, FIGS. 5A and 5B illustrate analyte sensor configuration including a sensing layer dimensioned to be at least as large as or larger than at least a portion of the conductive layer 504 of the working electrode. More specifically, referring to FIGS. 5A and 5B, sensor 500 in one embodiment includes a substrate 502 having a conductive layer 504 extending along at least a portion of the length of the substrate 502 to form the working electrode of the sensor. Conductive layer 504 may include a proximal portion and distal portion where the portions may be the same or different sizes and/or shapes, for example may include a narrow proximal portion 504a. which extends the length of substrate 502 and terminates in a wider or larger distal portion 504b having a width or diameter dimension W_{C}.

In certain embodiments, conductive layer 504 may be manufactured with a substantially constant width over the entire length, may have a wider proximal portion and a narrower distal portion, or the like. Distal portion 504b may have any suitable shape, including but not limited to circular (as illustrated), oval, rectilinear, or other equivalent shapes. Disposed over distal portion 504b of conducting layer 504 is a sensing layer 506. Again, sensing layer 506 may have any suitable shape and area dimension and may cover part of or the entirety of distal portion 504b of the conductive material. As shown in the Figures, sensing layer 506 in one aspect has substantially the same circular shape as that of distal portion 504b and an area having a width/diameter dimension W_{S} which is larger than (or at least as large as) that of distal portion 504b such that a peripheral border extends beyond the outer edge of distal portion 504b.

Irrespective of the area of the sensing material 506, the active area 510 of the sensor may be determined by the area of distal conductive portion 504b. In this manner, the dimension of the active area 510 may be varied by varying the area of the distal portion 504b of the conductive layer 504. Depending upon the dimensions of the conductive layer 504 for the working electrode, the area of a corresponding sensing layer may vary, but as shown, the sensing layer has an active area that is at least as large as the area of the corresponding conductive layer 504 forming the working electrode, as described above.

In certain embodiments, the width diameter of the sensing layer Ws may be in the range from about 0.05 mm to about 1.0 mm, e.g., from about 0.1 mm to about 0.6 mm, and the width/diameter of the conducting layer Wc is in the range from about 0.1 mm to about 1.0 mm, e.g., from about 0.2 mm to about 0.6 mm, with the resulting active area in the range from about 0.0025 mm² to about 1.0 mm², *e.g*., from about 0.01 mm² to about 0.36 mm².

Referring still to FIGS. 5A and 5B, in certain embodiments, an insulation/dielectric layer 508 is disposed or layered on at least a portion of proximal portion 504a of conducting layer 504. Additional conducting and dielectric layers may be provided.

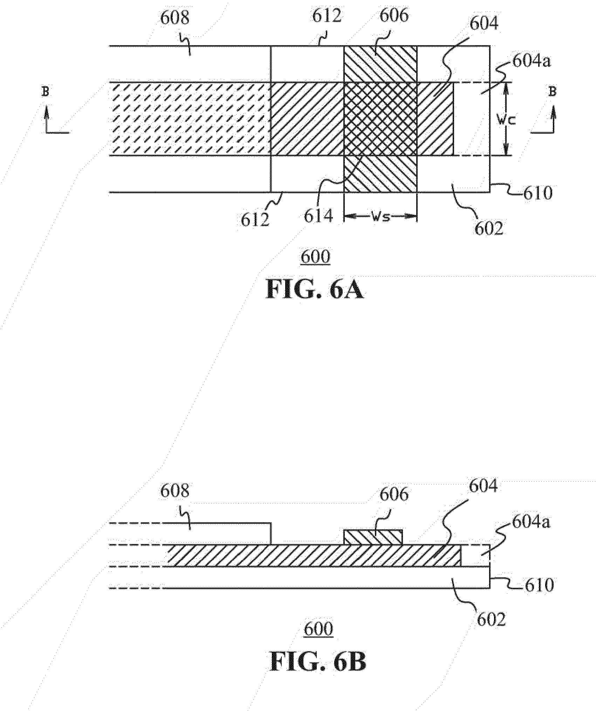

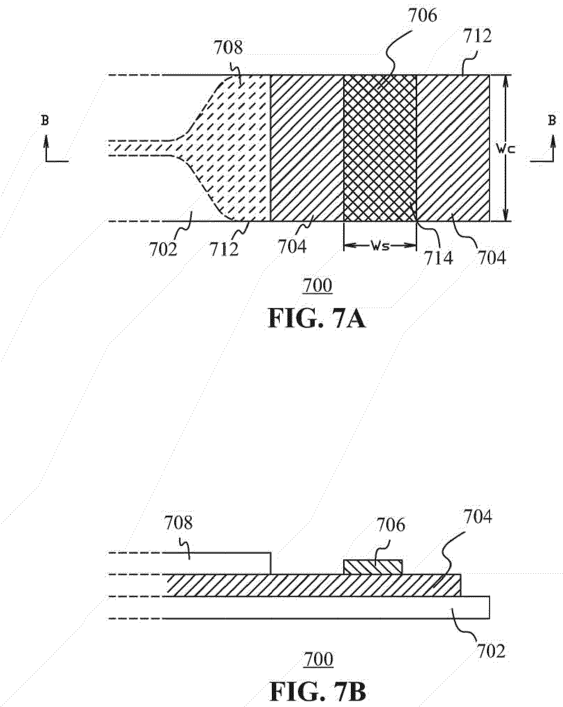

FIGS. 6A and 7A illustrate top views of an insertion tip or tail portion of respective sensors having precisely formed active areas, while FIGS. 6B and 7B are cross-sectional side views of the respective sensors taken along lines B-B of the respective FIGS. 6A and 7A. Referring now to FIGS. 6A and 6B, sensor 600 includes a substrate 602 having a conductive layer 604 extending along at least a portion of the length of the substrate to form the sensor's working electrode. Conductive layer 604 may terminate proximally of the distal edge 610 of substrate 602 and, as such, provides a "finger" configuration. Alternatively, the conductive layer 604a may extend to distal edge 61 0 of the sensor 600 as shown in the Figures. In one aspect, working electrode 604 has a width W_{C} which is less than the width of substrate 602, extending a selected distance from the side edges 612 of the substrate, which distance may be equidistant or vary from each of the side edges 612. Disposed over a portion of the length of conducting layer 604 is sensing layer 606 which, as shown in this embodiment, is provided in a continuous stripe/band substantially orthogonal to and extending from side edge 612 to side edge 612 of substrate 602. Sensing layer 606 has a width Ws, which may cover part or the whole length of the working electrode 604. As shown, the active area 614 is defined by the overlap of the working electrode 604 and the sensing layer 606.

Referring to FIGS. 6A and 6B, in certain aspects of the present disclosure, the width of the sensing layer W_{S} may be in the range from about 0.05 mm to about 5 mm, *e.g*., from about 0.1 mm to about 3 mm, and the width of the conductive layer W_{C} may be in the range from about 0.05 mm to about 0.6 mm, e.g., from about 0.1 mm to about 0.3 mm, with the resulting active area in the range from about 0.0025 mm² to about 3 mm², *e.g.,* from about 0.01 mm² to about 0.9 mm².

The orthogonal relationship between sensing layer 606 and conducting layer 604 provide the intersecting or overlapping portions resulting in the active area 614 having a rectilinear polygon configuration. However, within the scope of the present disclosure, any suitable shape of the active area may be formed or provided. The dimensions of the active area 614 may be varied by varying either or both of the respective width dimensions of the sensing and conducting layers. Referring back to the Figures, an insulation dielectric layer 608 is disposed or layered on at least a proximal portion of conducting layer 604.

Referring now to FIGS. 7A and 7B, in another embodiment, sensor 700 includes a substrate 702 having a conductive layer 704 (which in certain embodiments may be the first of several conductive layers, each corresponding to the respective one of the working electrode, counter electrode, and the reference electrode), extending along the length of the substrate to form the working electrode of the sensor 700. In embodiments of the present disclosure, the conductive layer 704 for the respective electrodes may be provided on the same plane over the substrate 702 such that the conductive layers for each of the working electrode, the counter electrode and the reference electrode are positioned or provided side by side on the substrate 702. In one aspect, the conductive layer 704 which forms the working electrode extends at least a portion of the length of substrate 702 and has at least a distal portion having a width dimension W_{C} which is shown in this embodiment to extend the width of substrate 702.

Disposed over a portion of the length of conductive layer 704 is sensing layer 706 provided in a continuous stripe/band substantially orthogonal to and extending from side edge 712 to side edge 712 of substrate 702. In one aspect, sensing layer 706 may have a defined width W_{S} which is narrower than the width W_{C} of working electrode 704 (as well as the width of the substrate 702), but may be substantially the same or wider than the working electrode and/or substrate. In certain embodiments, the width of the sensing layer W_{S} may be in the range from about 0.05 mm to about 5 mm, e.g., from about 0.1 mm to about 3 mm, and the width of the conducting layer W_{C}, *i.e.,* the width of the substrate, is in the range from about 0. 1 mm to about 1 mm, *e.g*., from about 0.2 mm to about 0.5 mm, with the resulting active area in the range from about 0.005 mm² to about 5 mm², *e.g*., from about 0.02 mm² to about 1.5 mm².

Again, as shown in the Figures, the orthogonal relationship between sensing layer 706 and conductive layer 704 results in the intersecting or overlapping portions defining the active area 714 with a rectilinear polygon configuration. However, within the scope of the present disclosure, any suitable shape may be provided. The dimensions of the active area 714 may be varied by varying the width dimension W_{S} of the sensing layer and/or the width dimension of the substrate which, in this case, is the same as the width dimension W_{C} of the conducting layer. As further shown in the Figures, an insulation/dielectric layer 708 may be disposed or layered on at least a proximal portion of conductive layer 704.

In accordance with certain embodiments, analyte sensors having accurately defined active areas as described above are fabricated so that they are reproducible. More specifically, one approach includes providing, depositing, printing, or coating a stripe/band of the sensing components orthogonally to the length of a conductive layer, typically the conductive layer which functions as the working electrode. This process may be performed before singulating/cutting the sensor from the sheet or web. In particular, if the fabrication process is web based, deposition of the sensing layer material is provided in a continuous process (striping) over adjacent sensors. The "sensing stripe" may be provided in a manner such that it has a constant width at least over the entire width of the conductive layer of a single sensor where the width dimension of the sensing stripe is orthogonal to the width dimension of the conductive material.

The length of the sensing material may extend beyond one or both of the edges of the width of the conductive layer. In certain aspects, the portion of the conductive layer upon which the sensing stripe is provided also has a constant width which may extend over the entire width of the sensor's substrate (FIG. 7A) or terminate or recede proximally of one or both of the substrate's side edges (FIG. 6A). The length of the conductive layer may extend the full length of the sensor to the distal edge of the sensor's substrate (Fig. 7A) or may be truncated at a defined distance proximal from the substrate's distal edge (Fig. 6A), with the latter configuration referred to as a "finger" construct.

With both the sensing and conductive layers/stripes having substantially constant widths and provided substantially orthogonal to each other, the active area which their intersection forms is also substantially constant along both the length and width of the sensor. In such embodiments, the active area has a rectilinear polygonal shape which may be easier to provide in a reproducible manner from sensor to sensor.

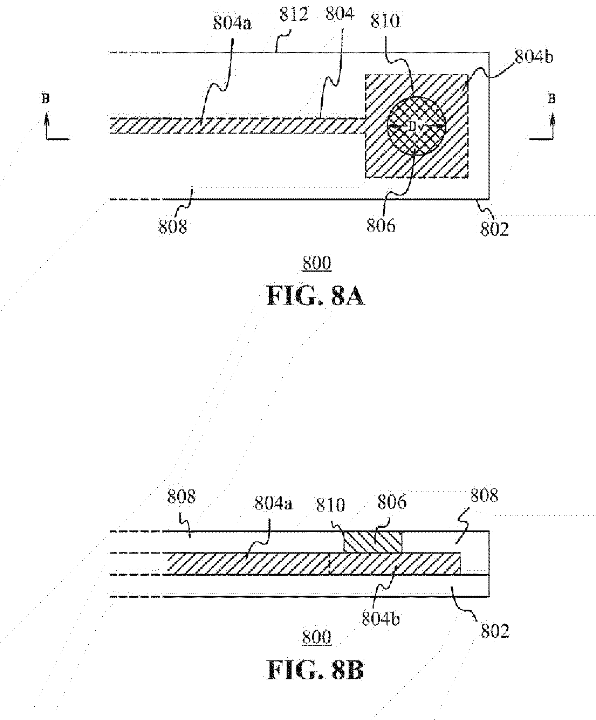

FIGS. 8A and 8B illustrate a top planar view and a cross sectional view respectively, of an analyte sensor according to the invention where the active area of the sensor is defined by a void or well within the dielectric layer (e.g., coverlay) over the sensor electrode (e.g., the working electrode), and which is filled with the sensing components. Referring to the Figures, in one embodiment, sensor 800 includes a substrate 802 having a conductive layer 804 extending along a portion of the length of the substrate to form the working electrode of the sensor. Conductive layer 804 may include a narrow proximal portion 804a, which extends the majority of the length of substrate 802 and terminates in a wider or larger distal portion 804b having a width or diameter dimension W_{C}. In certain aspects, conductive layer 804 may have a substantially constant width over its entire length, may have a wider proximal portion and a narrower distal portion, and the like. Distal portion 804b may have any suitable shape, including but not limited to rectilinear, oval, circular, or other equivalent shapes. Disposed over conducting layer 804 is a dielectric layer 808 as shown in Fig. 8B having a void or well 810 therein which is positioned over the distal portion 804b of the conducting layer 804. While dielectric layer 808 is also shown overlaying substrate 802a to its peripheral edges 812, the outer periphery of dielectric layer 808 may have any suitable boundary. Disposed within void 810 is the sensing material 806, which defines the active area of the sensor. Embodiments further include a glucose flux limiting layer, an interference layer, a biocompatible layer, or the like, that may be disposed in or on top of void 810. For example, embodiments include dielectric layer 808 that is sized to approximate the dimensions of a void/well and not layered over other portions of the sensor 800.

Referring back to FIGS. 8A and 8B, the side wall(s) of void well 810, and thus the shape of the active area 806 of the sensor, may have any suitable shape, including but not limited to circular (as illustrated), oval, rectilinear and the like. The area dimension of void 810 is determined based on the diameter dimension D_{V} (in the case of circular voids) or width and length dimensions (in the case of rectilinear voids) is selected based on the desired area of the active area 806 of the sensor. Thus, the dimension of the active area 806 may be varied by varying the area of void 810 during the fabrication process. In addition, the defined and reproducible void/well 810 in one embodiment as shown in FIGS. 8A and 8B may define the thickness of the glucose limiting membrane of the *in vivo* sensor. For example, referring back to FIG. 3B, in one embodiment, the portion of the coverlay material 118 that is removed to define or expose a predetermined active sensing area on the working electrode 121a may further define the thickness of the glucose limiting membrane that is disposed over the active sensing area 112.

While the area of the void 810 is illustrated as being smaller than that that of conductive distal portion 804b, in certain embodiments, it may be as large as the latter area, but in certain embodiments not larger. Additionally, while void 810/active area 806 is illustrated as being centrally disposed within the area of conductive distal portion 804b, within the scope of the present disclosure, the position of the void 810/active area 806 may not be centered but rather offset within the area of the conductive distal portion 804b. In certain embodiments, the area of the active area is in the range from about 0.01 mm² to about 1.0 mm², *e.g*., from about 0.04 mm² to about 0.36 mm².

As the active area in the embodiment of FIG. 8A and 8B is dependent on the area of the void 810 within dielectric material 808, fabrication techniques using a dielectric material supports a high degree of precision application as well as precision techniques for applying the dielectric material and forming the void therein are provided. For example, photo-imageable polymeric materials may be used for the dielectric material which is deposited on the substrate/conductive material from solution or by roll press process using the photo-imageable film and the void formed therein by a photolithographic process.

### Precise Sensor Dimension

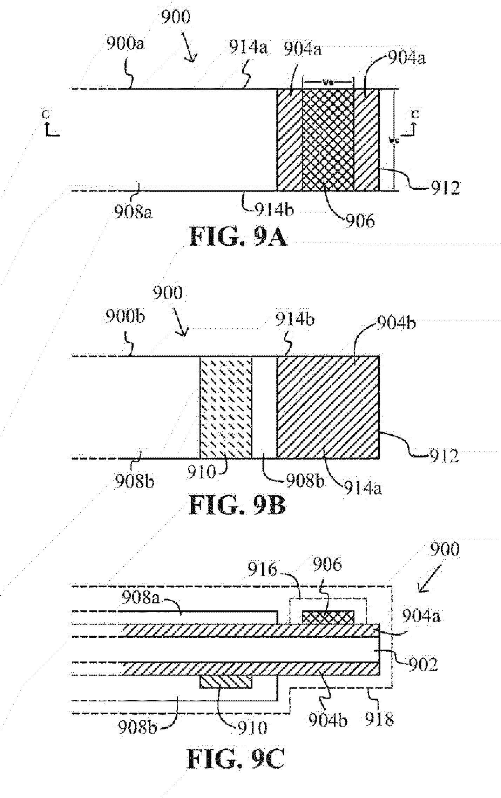

FIGS. 9A-9C illustrate top, bottom and cross sectional side views, respectively of a two sided analyte sensor in accordance with one aspect in which two sides of a dielectric include conductive material. Referring to FIGS. 9A-9C, an embodiment of a double-sided implantable portion of the sensor 900, *e.g.,* the distal portion of the sensor's tail section, is illustrated. In particular, FIGS. 9A and 9B provide top and bottom views, respectively, of tail section 900 and FIG. 9C provides a cross-sectional side view of the same taken along lines C-C in FIG. 9A.

Referring to the Figures, in one aspect, sensor tail portion 900 includes a substrate 902 (FIG. 9C) having a top conductive layer 904a which substantially covers the entirety of the top surface area of substrate 902. That is, the conductive layer 904a substantially extends the entire length of the substrate to distal edge 912 and across the entire width of the substrate from side edge 914a to side edge 914b. Similarly, the bottom conductive layer 904b substantially covers the entirety of the bottom side of the substrate of tail portion 900. As further shown, one or both of the conductive layers may terminate proximally of distal edge 912 and/or may have a width which is less than that of substrate 902 where the width terminates a selected distance from the side edges 914a, 914b of the substrate, which distance may be equidistant or vary from each of the side edges.

In one aspect, one of the top or bottom conductive layers, here, top conductive layer 904a, may be configured to function as the working electrode of the sensor while the opposing conductive layer - bottom conductive layer 904b - is configured as a reference and/or counter electrode. In certain embodiments, a working electrode may be positioned on both sides of a sensor to provide a single sensor with two working electrodes. In embodiments with conductive layer 904b configured as either a reference or counter electrode, but not both, a third electrode may optionally be provided on a surface area of the proximal portion of the sensor (not shown). For example, conductive layer 904b may be configured as a reference electrode and a third conductive layer (not shown), present on the non-implantable proximal portion of the sensor, may function as the counter electrode of the sensor.

Referring back to the Figures, disposed over a distal portion of the length of conducting layer/working electrode 904a is sensing component 906. As only a small amount of sensing material is required to facilitate oxidization or reduction of the analyte, positioning the sensing layer 906 at or near the distal tip of the sensor tail reduces the amount of material needed. Sensing layer 906 may be provided in a continuous stripe/band between and substantially orthogonal to the substrate's side edges 914a, 914b with the overlap or intersection of working electrode 904a and the sensing layer 906 defining the active area of the sensor. Due to the orthogonal relationship between sensing layer 906 and conducting layer 904, the active area has a rectilinear polygon configuration. However, any suitable shape may be provided. The dimensions of the active area 914 may be varied by varying either or both of the respective width dimensions of the sensing and conducting layers. The width W_{S} of the sensing layer 906 may cover the entire length of the working electrode or only a portion thereof. As the width W_{C} of the conductive layer is governed by the substrate width of the tail portion in this embodiment, any registration or resolution inconsistencies between the conductive layer and the substrate are obviated. In certain embodiments, the width of the sensing layer W_{S} is in the range from about 0.05 mm to about 5 mm, *e.g*., from about 0.1 mm to about 3 mm; the width of the conductive layer W_{C} is in the range from about 0.05 mm to about 0.6 mm, e.g., from about 0.1 mm to about 0.3 mm, with the resulting active area in the range from about 0.0025 mm² to about 3 mm², *e.g.,* from about 0.01 mm² to about 0.9 mm².

Referring again to the electrodes, in certain embodiments, the same materials and methods may be used to fabricate the top and bottom electrodes, although different materials and methods may also be used. With the working and reference electrodes positioned on opposing sides of the substrate as in the illustrated embodiment of FIGS. 9A-9C, in certain embodiments, two or more different types of conductive material to form the respective electrodes may be used.

Selection of the conductive materials for the respective electrodes is based in part on the desired rate of reaction of the sensing layer's mediator at the sensor electrode. In certain embodiments, the rate of reaction for the redox mediator at the counter/reference electrode is controlled by, for example, selecting a material for the counter/reference electrode that would require an overpotential or a potential higher than the applied potential to increase the reaction rate at the counter/ reference electrode. For example, some redox mediators may react faster at a carbon electrode than at a silver/silver chloride (Ag/AgCl) or gold electrode.

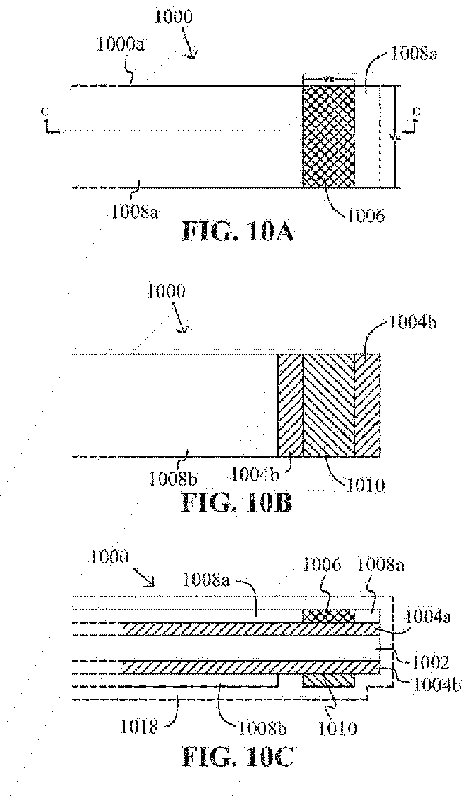

Accordingly, in certain aspects, the sensor embodiment shown in FIGS. 9A-9C provides a sensor construct including substantially full-length conductive layers 904a, 904b that includes materials such titanium, gold carbon or other suitable materials with a secondary layer of conductive layer 910 of a material such Ag/AgCl disposed over a distal portion of bottom conductive layer 904b to collectively form the reference electrode of the sensor. As with sensing layer 906, conductive material 910 may be provided in a continuous stripe/band between and substantially orthogonal to the substrate's side edges 914a, 914b. While layer 910 is shown positioned on substrate 902 proximally of sensing layer 906 (but on the opposite side of the substrate), layer 910 may be positioned at any suitable location on the tail portion 900 of the reference electrode 904a. For example, as illustrated in FIGS. 10A-10C, the secondary conductive material 1010 of reference electrode 1008b may be aligned with and/or distal to sensing layer 1006.

Referring again to the Figures, an insulation/dielectric layer 908a, 908b may be disposed on each side of the sensor 900 over at least the sensor's body portion (not shown), to insulate the proximal portion of the electrodes, *i.e.,* the portion of the electrodes which in part remains external to the skin upon transcutaneous positioning. The upper dielectric layer 908a disposed on the working electrode 904a may extend distally to but not over any portion of sensing layer 906, or in certain embodiment may cover some but not all of sensing layer 906. Alternatively, as illustrated in FIGS. 10A-10C, dielectric layer 1008a on the working electrode side of the sensor may be provided prior to sensing layer 1006 such that the dielectric layer 1008a has at least two portions spaced apart from each other on conductive layer 1004a, best illustrated in FIG. 10C. The sensing material 1006 is then provided in the spacing between the two portions.

As for the dielectric layer on the bottom/reference electrode side of the sensor, it may extend any suitable length of the sensor's tail section, *i.e.*, it may extend the entire length of both of the primary and secondary conductive layers or portions thereof. For example, as illustrated in FIGS. 10A-10C, bottom dielectric layer 1008b extends over the entire bottom surface area of secondary conductive material 1010 but terminates proximally of the distal edge 1012 of the length of the primary conductive layer 1004b. It is noted that at least the ends of the secondary conductive material 1010 which extend along the side edges substrate 1002, while initially covered by dielectric layer 1008b, after singulation of the sensors, the secondary conductive layer 1010 is exposed along the side edges of the substrate 1002 and, as such, are exposed to the *in vivo* environment when in operative use. As further illustrated in FIGS. 10A-10C, bottom dielectric layer 1008b in certain embodiments may have a length which terminates proximally of secondary conductive layer 1010.

Additionally, one or more membranes which may function as one or more of an analyte flux modulating layer and/or an interferent-eliminating layer and/or biocompatible layer may be provided about the sensor as one or more of the outermost layer(s). In certain embodiments, as illustrated in FIG. 9C, a first membrane layer 916 may be provided solely over the sensing component 906 on the working electrode 904a to modulate the rate of diffusion or flux of the analyte to the sensing layer. For embodiments in which a membrane layer is provided over a single component/material, it may be suitable to do so with the same striping configuration and method as used for the other materials/components. Here, the stripe/band of membrane material 916 may have a width greater than that of sensing stripe/band 906.

As it acts to limit the flux of the analyte to the sensor's active area, and thus contributes to the sensitivity of the sensor, controlling the thickness of membrane 916 is important. That is, fabrication of reproducible analyte sensors includes substantially constant membrane thickness. Providing membrane 916 in the form of a stripe/band facilitates control of its thickness. A second membrane layer 918 which coats the remaining surface area of the sensor tail may also be provided to serve as a biocompatible conformal coating and provide smooth edges over the entirety of the sensor. In other embodiments, as illustrated in FIG. 10C, a single, homogenous membrane 1018 may be coated over the entire sensor surface area, or at least over both sides of the distal tail portion. It is noted that to coat the distal and side edges of the sensor, the membrane material would have to be applied subsequent to singulation of the sensor precursors.

In certain embodiments, the membrane coating with high precision over the sensor lot may be achieved in several ways. In the case where the membrane is applied after the sensor singulation process, the membrane may be applied by spray coating or dipping, for example. In the case of dipping, control over the viscosity of the membrane formulation over the course of the sensor lot is be controlled by, for example, reducing the temperature of the dip bath. Alternatively, a sensor may be incorporated into the dip bath where the viscosity can be directly determined and dipping parameters such as exit speed can be controlled to account for changing viscosity over the course of the sensor lot, keeping the dipped thickness substantially the same regardless of potential in-process variation of the raw components (*e.g*., sensor composition materials).

In certain embodiments, other detectors or measurement devices or systems may be used to monitor the thickness of the membrane application and adjust the process parameters to ensure low thickness variability over the course of the sensor lot. For example, the detectors or measurement devices or systems may be selected from for example, laser displacement detectors, confocal laser displacement detectors, including those that operate at short wavelengths, capacitive detectors, and other detectors or measurement devices that can measure, detect or determine one or more of the thickness of the membrane and/or the underlying electrode such that, based on the measured or detected information, adjustment to the sensor lot may be made to maintain low thickness variability resulting in minimal or insignificant sensor to sensor variation within each sensor lot during manufacturing. In aspects of the present disclosure, the aforementioned measurement or detection of the membrane thickness may be performed for each sensor, and sensor(s) with a membrane thickness measured or determined that is outside a thickness tolerance range (as defined or determined based on a tolerance criteria for variation between the sensors) may be discarded during the manufacturing process, or tagged or flagged as unsuitable for *in vivo* use.

### Sensor Fabrication Process - Two sided sensor

Improving upon the accuracy of providing the sensing component on the sensor, and thus, the accuracy of the resulting active area, may significantly decrease any sensor to sensor sensitivity variability and obviate the need for calibration of the sensor during *in vivo* use. Additionally, the methods provide finished sensors which are smaller than currently available sensors with micro-dimensioned tail portions which are far less susceptible to the *in situ* environmental conditions which can cause spurious low readings.

In a variation of the subject methods, web-based manufacturing techniques are used to perform one or more steps in fabricating the subject sensors, many of the steps of which are disclosed in U.S. Patent No. 6,103,033. To initiate the fabrication process, a continuous film or web of substrate material is provided and heat treated as necessary. The web may have precuts or perforations defining the individual sensor precursors. The various conductive layers are then formed on the substrate web by one or more of a variety of techniques as described above, with the working and reference (or counter/ reference) electrode traces provided on opposite sides of the web.

Also, as mentioned previously, a third, optional electrode trace (which may function as a counter electrode, for example) may be provided on the proximal body portion of the sensor precursors. The "primary" conductive traces provided on the area of the tail portions of the precursor sensors have a width dimension greater than the desired or predetermined width dimension of the tail portions of the final sensor configuration. The precursor widths of the conductive traces may range from about 0.3 mm to about 10 mm including widths in range from about 0.5 mm to about 3 mm, or may be narrower, e.g., from about 2 mm to about 3 mm. In certain embodiments, the primary conductive layers may be formed extending distally along the tail section of the sensor precursors to any suitable length, but preferably extend at least to the intended distal edge of the finalized sensors to minimize the necessary sensor tail length.

Next, the sensing layer and secondary conductive layers, if employed, are formed on the primary conductive layers on the respective sides of the substrates or substrate web. As discussed, each of these layers may be formed in a stripe or band of the respective material disposed orthogonally to the length of the primary conductive layer/sensor tail. With a single, continuous deposition process, the mean width of the sensing strip is substantially constant along the substrate webbing, and ultimately, from sensor to sensor. The secondary conductive layer (*e.g.*, Ag/AgCl on the reference electrode), if provided, may also be formed in a continuous orthogonal stripe/band with similar techniques. One method of providing the various stripes/bands of material on the sensors is by depositing, printing or coating the sensing component/material by means of an inkjet printing process (e.g., piezoelectric inkjet as manufactured by Scienion Inc. and distributed by BioDot Inc.). Another way of applying these materials is by means of a high precision pump (*e.g.*, those which are piston driven or driven by peristaltic motion) and/or footed needle, as described in further detail in application no. 61/165,488 titled "Precise Fluid Dispending Method and Device". The respective stripes/bands may be provided over a webbing of sequentially aligned sensor precursors prior to singulation of the sensors or over a plurality of sensors/electrodes where the sensors have been singulated from each other prior to provision of the one or more stripes/bands.

With both the sensing and conductive layers/stripes having substantially constant widths and provided substantially orthogonal to each other, the active area which their intersection forms is also substantially constant along both the length and width of the sensor. In such embodiments, the active area (as well as the intersecting area of the primary and secondary conductive layers which form the reference electrode) has a rectilinear polygonal shape which may be easier to provide in a reproducible manner from sensor to sensor, however, any relative arrangement of the layers resulting in any suitable active area geometry may be employed.

The sensor precursors, *i.e.,* the template of substrate material (as well as the conductive and sensing materials if provided on the substrate at the time of singulation), may be singulated from each other using any convenient cutting or separation protocol, including slitting, shearing, punching, laser singulation, etc. These cutting methods are also very precise, further ensuring that the sensor's active area, when dependent in part on the width of the sensor (*i.e.*, the tail portion of the substrate), has very accurate dimensions from sensor to sensor. Moreover, with each of the materials (i.e., the primary and secondary conductive materials, sensing component, dielectric material, membrane, etc.) provided with width and/or length dimensions extending beyond the intended dimensions or boundaries of the final sensor units, issues with resolution and registration of the materials is minimized if not obviated altogether.

The final, singulated, double-sided sensor structures have dimensions in the following ranges: widths from about 600 µm to about 100 µm, including widths in range from about 400 µm to about 150 µm; tail lengths from about 10mm to about 3 mm, including lengths in range from about 6 mm to about 4 mm; and thicknesses from about 500 pin to about 100 µm, including thicknesses in range from about 300 µm to about 150 µm. As such, the implantable portions of the sensors are reduced in size from conventional sensors by approximately 20% to about 80% in width as well as in cross-section. The reduced size minimizes bleeding and thrombus formation upon implantation of the sensor and impingement on adjacent tissue and vessels, and thereby minimizes impediment to lateral diffusion of the analyte to the sensor's sensing component.

### Sensor Fabrication Processes

As discussed, at least one factor in minimizing variations in sensor sensitivity within the same sensor batch or lot (or with all sensors made according to the same specification) may include maintaining the dimensions (such as area, width, length, and/or diameter) of the active area from sensor to sensor. Accordingly, aspects of the present disclosure include analyte sensors having accurately defined active areas. This accuracy is achieved by maintaining substantially the same geometry/shape and dimensions of the sensing layer. In current practice, the methods of applying the sensing layer (e.g., by means of an ink jet printing process or by means of a high precision pump and/or footed needle) result in significant variations in the geometry/shape and dimensions of the sensing layer.

In certain embodiments, methods and processes for fabricating analyte sensors with active areas that are substantially identical sensor to sensor are provided. Certain aspects include removing a portion of the sensing layer and/or conductive layer to attain the desired dimensions and surface area of the intended active area. Any suitable subtractive process may be employed to remove the targeted material method. One such process includes using a laser to ablate away or trim the targeted material.

Generally, a laser ablation system includes a power supply (e.g., with a pulse generator), lasing medium, and a beam delivery subsystem. The power supply pulse generator if employed generates a pulsed laser output at a selected pulse repetition rate. The beam delivery subsystem includes at least one beam deflector to position the laser pulses relative to the material to be trimmed, and the optical subsystem focuses the laser pulses into a spot within a field of the optical subsystem.

Beam delivery systems for fabrication of high precision analyte sensors in certain aspects include scanner systems (scan head systems) that include one or more moving mirrors which steer a laser beam delivered into the scanner through a fixed working area. Such scanner system may include a flat field objective lens (f-theta lens) that serves to focus the beam onto a planar surface. Alternately, high speed focusing optics such as a VarioScan (ScanLab, Germany) may be used to focus the beam in a three dimensional space. A further configuration may use a scanner moving in one or more axes coupled to a motion platform that moves the part in one or more axes, for example, perpendicular to the at least one scanner axis. The second axis may move independently or in a coordinated manner made possible computer numerical control (CNC) where the scanner moves in concert with the motion system to fabricate the part.

Another beam delivery system includes a fixed beam delivery system where the part is moved in typically X, Y and or theta and the optics remain fixed. In another aspect, the fixed beam system may be configured to move in one or more axes relative to the stage that holds that part to be machined that moves in one or more axes, for example perpendicular to the first axis. Also, a combination of the fixed beam delivery system and the scanner system described above may be used.

In still another aspect, a mask projection system may be used to remove material through the open areas of the mask. Each laser pulse has pulse energy, a laser wavelength, a pulse width, a frequency (or repetition rate) and a spot diameter. These parameters are selected based on the type, density and thickness of the targeted material(s), as well as the size of the element, area, or layer of material(s) to be removed, trimmed. In the sensor fabrication applications of the present invention, the selected wavelength is short enough to produce desired short-wavelength benefits of small spot size, tight tolerance, high absorption, and reduced or eliminated heat-affected zone (HAZ) along the trim path.

In one aspect, an ultraviolet (UV) laser is employed to trim or ablate the excess material. UV lasers for use in the manufacturing process may include lasers with ultraviolet wavelengths below 400 nm, such as excimer lasers and diode pumped solid state lasers with third and fourth harmonics. In certain embodiments, UV wavelengths ranging from about 10 nm to about 380 nm are employed. In a particular embodiment, the wavelength of the UV laser used is shorter than about 355 nm, and more specifically, in the range from about 266 nm to about 355 nm. Because of the relatively shorter wave lengths employed, ablation of the targeted material occurs by a photochemical reaction rather than by a thermal reaction. As the ablation is accompanied by substantially no heat transfer or thermal shock, it does not cause serious damage, such as cracking, to the material being ablated or to any of the underlying layers or substrate material. As such, this type of ablation is often referred to as "cold ablation". Also, with cold ablation, the ablated surface is substantially free from re-deposited or resolidified material.

In certain embodiments, a laser having a pulse width of shorter than about 100 nano (10⁻⁹) seconds (ns) and a repetition rate from about 20 to about 80 kilohertz (KHz) can be used to fabricate these sensors. In one particular embodiment of the present invention, laser ablation may be conducted with an ultra fast laser. "Ultrafast lasers" refer to lasers consisting of pulses with durations shorter than about 10 pico (10⁻¹²) second (ps) and into the femto (10⁻¹⁵) second (fs) range. These lasers ablate using a multiphoton mechanism that differs from the single photon ablation mechanism used by UV lasers. As such, the requirements of linear optical absorption do not apply to ultrafast lasers which can use wavelengths throughout the UV and near infrared (IR) spectrum. An example of an ultrafast industrial laser suitable for this process is the 1552 nm laser made by Raydiance in Petaluma, CA having pulse widths of 800 fs and repetition rates of up to approximately 200 KHz.

Examples of UV lasers for use in conjunction with the fabrication process for the analyte sensors in accordance with aspects of the present disclosure include a neodymium YAG (Nd:YAG) (1064 nm) laser such as a diode pumped solid state laser, a YAG laser with a third or fourth harmonic generation package, a XeF excimer laser, an argon fluoride (ArF) laser having 193 nm wavelength, and a fluorine (F₂) laser having 152 nm wavelength. In particular, excimer lasers commercially available from Coherent, Inc., located in Santa Clara, CA, which are integrated into machines from suppliers, such as Photomachining of Pelliam NH, Tamarack Scientific of Los Angeles, CA, Resonetics Corporation of Nashua, New Hampshire, and Exitech Limited of Oxford, England, may also be used.

In further aspects, a fiber or diode pumped solid state laser having a 1064 nm wavelength may be used to trim or ablate the excess material during the sensor manufacturing process.

The intensity (fluence) of the laser radiation that is required to trim a material is dependent on the material to be ablated. By adjusting the intensity of the laser, it is possible to ablate the entire thickness of the sensing material without ablating the electrode material, or, as the case may be, ablating both the sensing and conductive material without ablating the substrate. Alternatively, the thickness of the coating may be estimated before ablation, and the intensity and/or pulse number of the laser can be adjusted to properly ablate the estimated thickness. Specifically, each material has its own laser-induced optical breakdown (LIOB) threshold which characterizes the fluence required to ablate the material at a particular pulse width. Also the fluence of the laser suitable for the present invention can be chosen according to the thickness of the layer or layers targeted for ablation. Furthermore, the number of pulses needed to ablate completely through a material can be calculated for a given energy or fluence. In other words, a laser may be employed having an appropriate intensity to trim one or more targeted or selected layers without ablating one or more of the underlying layers. For example, a UV laser may be adjusted to trim a sensor's sensing layer without ablating the underlying conductive layer or any intervening layers, if any. Or, by further example, the laser may be adjusted to trim to a depth or thickness of both the sensing and conductive layers but not below the conductive layer.

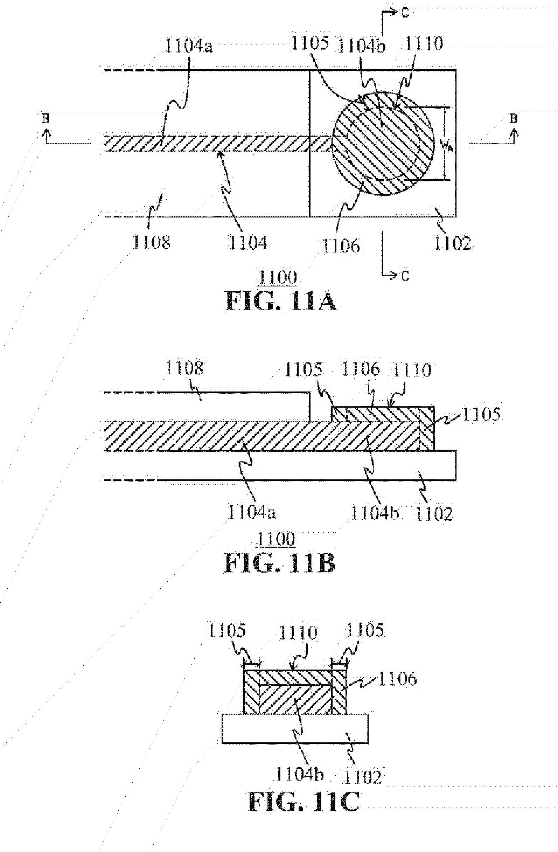

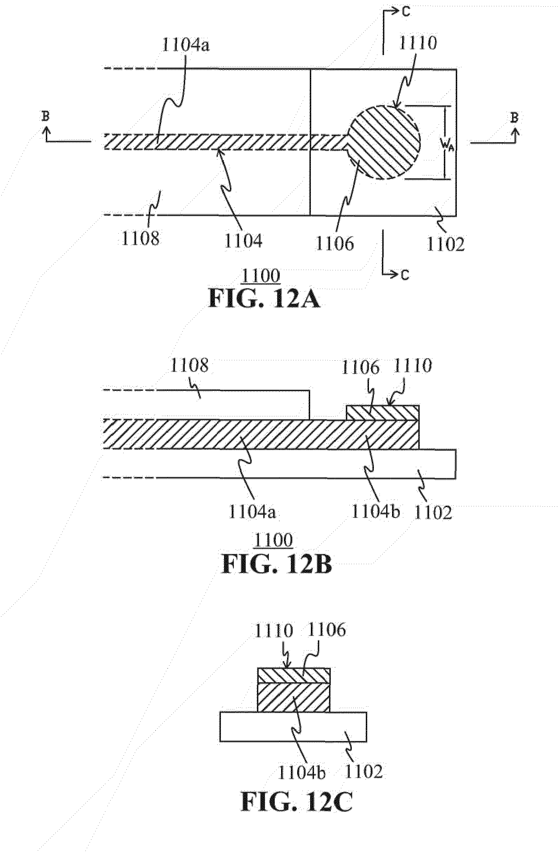

In one aspect, material from the sensing layer is removed such that the surface area dimensions and/or geometry/shape of the sensing layer match the surface area dimensions and/or geometry/shape of the underlying conductive material of the working electrode. In another aspect, where both the dimensions of the conductive material and the sensing material extend beyond the perimeter of the intended surface area of the sensor active area, portions of both layers may be ablated/trimmed to the desired dimensions. Yet another aspect includes only removing a small portion or a wedge of the sensing layer and the underlying conductive layer to affect the desired active area. Each of the three exemplary sensors described below are first illustrated in a pre-ablation or pre-trim configuration (see FIGS. 11A-11C, 13A-13C, and 15A-15C, respectively) and then in a post-ablation or post-trim configuration (see FIGS. 12A-12C, 14A-14C and 16A-16C, respectively).

Referring in particular to FIGS. 11A-11C and FIGS. 12A-12C, the illustrated sensor 1100 includes a substrate 1102 having a conductive layer 1104 extending along at least a portion of the length of the substrate to form the sensor's working electrode. Conductive layer 1104 includes a narrow proximal portion 1104a which extends the majority of the length of substrate 1102 and terminates in a wider or larger distal portion 1104b having a width or diameter dimension W_{A}. In certain aspects, conductive layer 1104 may have a constant width over its entire length, or may have a wider proximal portion and a narrower distal portion. Distal portion 1104b may have any suitable shape, including but not limited to circular (as illustrated), oval, rectilinear, of other appropriate shapes.

In this embodiment, only the distal portion 1104b is intended to define the surface area dimensions (width/length or diameter) of the active area of the sensor. That is, W_{A} defines the desired width or diameter, of the intended active area 1110 (FIGS. 12A-12C). Deposited over distal portion 1104b of conducting layer 1104 is a sensing layer 1106. Preferably, sensing layer 1106 is provided with a shape or has a geometry and surface area which exactly or substantially exactly equal to or matches the geometry and dimensions of the underlying conductive layer 1104. This may be verified automatically by means of a computer-controlled digital camera or by visual inspection with a microscope.

However, should an excess amount of the sensing material 1 106 be provided such that its border or perimeter extends beyond that of the underlying conductive layer 1104, whether wholly or in part, as shown in FIGS. 11A-11C, the excess material margin 1105 may be trimmed by the laser process described above to provide the desired active area 1110 shape and dimensions, as illustrated in FIGS. 12A-12C. Sensor 1100 further includes an insulation or dielectric layer 1108 disposed or layered on at least a portion of proximal portion 1104a of conducting layer 1104. The insulation/dielectric layer 1108, as well as any additional conducting and dielectric layers, is typically provided prior to the above-described laser-trimming.

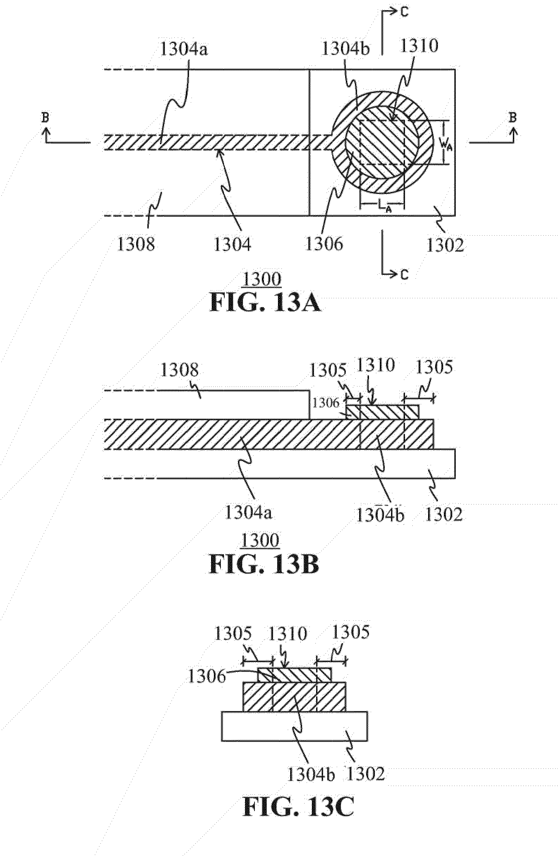

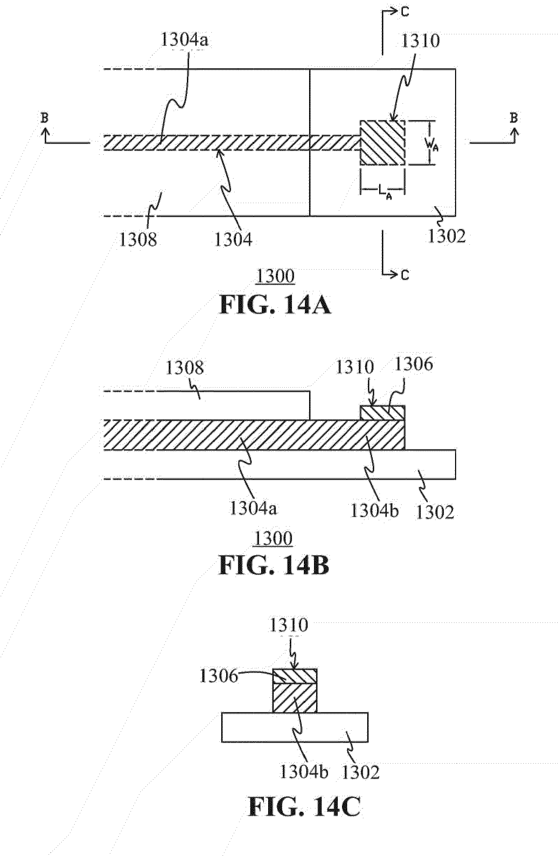

Another sensor fabricated according to the above described processes and techniques are illustrated in FIGS. 13A-13C in a pre-ablation configuration and in FIGS. 14A-14C in a post-ablation configuration. Sensor 1300 includes a substrate 1302 having a conductive layer 1304 (which may be the first of several conductive layers, one for each sensor electrode) extending along at least a portion of the length of the substrate to form the sensor's working electrode. Conductive layer 1304 has a similar configuration to that of conductive layer 1104 (FIGS. 11A-11C) described above (and any aforementioned variations thereof), having a narrow proximal portion 1304a which extends the majority of the length of substrate 1 302 and terminates in a wider or larger distal portion 1304b. However, as shown in FIGS. 13A and 14A, for example, distal portion 1304b is larger than the surface area (W_{A} × L_{A}) of the sensor's intended active area 1310 (FIGS. 14A-14C), which in this embodiment has a square or rectangular shape.

Deposited over distal portion 1304b of conducting layer 1304 is a sensing layer 1306. Unlike the larger, pre-trimmed sensing layer 1106 of FIGS. 11A-11C, sensing layer 1306 is smaller than the underlying conducting layer 1304, but still greater than the desired amount for the intended active area 1310. As such, the dimensions of the sensing layer 1306, as well as those of conducting layer 1304b, extend beyond the intended active area 1310. Employing the laser techniques of the embodiments of the present disclosure described above, the excess material margin 1305 may be trimmed or ablated to provide the desired active area 1310 shape and dimensions, as illustrated in FIGS. 14A-14C. Also shown is an insulation/dielectric layer 1308 that is disposed or layered on at least a portion of proximal portion 1304a of conducting layer 1304.

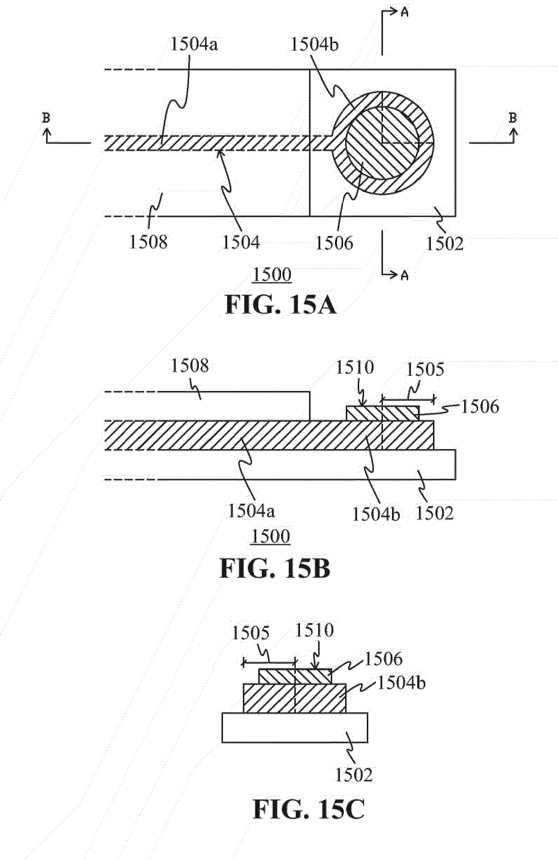

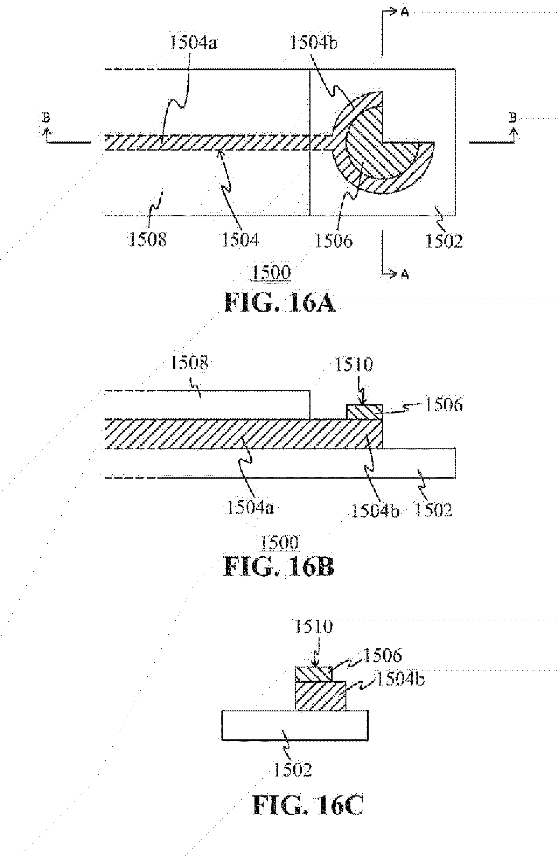

Another sensor fabricated according to the above described process and technique is illustrated in FIGS. 15A-15C in a pre-ablation configuration and in FIGS. 16A-16C in a post-ablation configuration. As shown, sensor 1500 includes a substrate 1502 having a conductive layer 1504 (which may be the first of several conductive layers, one for each sensor electrode) extending along at least a portion of the length of the substrate to form the sensor's working electrode. Conductive layer 1504 has a similar configuration to that of the conductive layers described above as well as the variations discussed, having a narrow proximal portion 1504a which extends the majority of the length of substrate 1502 and terminates in a wider or larger distal portion 1504b. Deposited over distal portion 1504b of conducting layer 1504 is a sensing layer 1506 which has a similar geometry but a smaller surface area than the underlying conducting layer 1504. As the size of the intended active area 1510 (FIGS. 16A-16C) is dependent upon the overlapping surface areas of the conductive material 1504 and the sensing material 1506, whether the conductive layer extends beyond the perimeter of the sensing layer or visa-versa may not be significant. As such, as long as each of the two layers has a surface area that is at least as great as the intended active area 1510, any excess material 1505 of one or both layers may trimmed or removed to provide a net overlapping surface area to provide the desired active area. In this embodiment, the surface area of both the conductive layer 1504 and sensing layer 1506 is greater than that of the intended surface area of the active area 1510 as illustrated in FIGS. 16A-16C,

Using the laser techniques described above, any excess material 1505 of either or both layers may be trimmed or ablated to provide the desired active area 1510 surface area, where the shape of the excess material 1505 to be removed may be any suitable shape to facilitate the trimming process. For example, as shown in FIGS. 15A and 1 6A, approximately a quarter of each of the layers has been trimmed by removing a piece or a wedge 1505 of the layers. In some embodiments, the excess material to be removed may be exclusively within the perimeter of both layers. If the shape of the particular laser cut is irrelevant, then it may be preferable to laser trim along the shortest necessary path. As with the above described sensor embodiments, an insulation/dielectric layer 1508 disposed or layered on at least a portion of proximal conducting portion 1504a. Additional conducting and dielectric layers may be provided as described herein.

In certain embodiments described, the diameter or width/length dimensions (W_{A}, L_{A}) of the desired active area is in the range from about 0.1 mm to about 1.0 mm, and preferably from about 0.2 mm to about 0.6 mm, with the resulting surface area in the range from about 0.05 mm² to about 0.5 mm², and preferably from about 0.08 mm² to about 0.15 mm².

As discussed above, in accordance with the various embodiments of the present disclosure, fabrication processes and procedures described herein provide well defined active area and substantially constant membrane dimensions (e.g., thickness) resulting in reproducible analyte sensors with minimal sensor to sensor sensitivity variations within the sensor lot or batch. Accordingly, minimal sensitivity variation in addition to a substantially stable shelf life profile provides obviates the need for sensor calibration during *in vivo* use. In certain embodiments, sensors within and/or between manufactured lots may be provided that have a coefficient of variation (CV) of about 5% or less, e.g., about 4.5% or less, e.g., about 4% or less, e.g. about 3% or less, where in certain embodiments CVs of between 1-3% are achieved.

### Sensor Packaging

Embodiments of the present disclosure includes packaging the *in vivo* analyte sensors such that the sensors are substantially impervious to the environmental effects of ambient air, particularly the effects of humidity, to which the sensors may be exposed prior to *in vivo* use, *i.e.,* during their shelf-life, in order to minimize any variation in the sensor characteristics, and degradation in their stability, and obviate the need for any user-based calibrations.

In aspects of the present disclosure, the subject sensors are individually packaged (but may be packaged in pairs or groups in removable packaging at the factory which packaging is not to be removed until the enclosed sensor is to be used, *i.e.*, implanted within a user's body. The removable packaging may include of one or more pieces, components or materials.

The packaging may include a two-piece housing structure having a tray and a lid or cover. The tray may have a relatively rigid construct to protect the sensor during shipping, handling, and storage over the course of the sensor's shelf-life. in one embodiment, the tray has an open portion thorough which the sensor is received and retrieved, and a closed or receptacle portion which provides a space or compartment within which the sensor is held. In one aspect, the tray has a shape and size which minimizes the unoccupied volume of the package in order to minimize the amount of air within the package as well as to minimize movement of the sensor within the packaging. Still yet, the tray may be contoured internally to match the shape of the sensor and any other packaged contents to eliminate any excess volume within the enclosed packaging. The tray may also be externally contoured to conform to other packaging or the like, and may have an outwardly extending edge or lip for engaging with a corresponding cover or lid.

In one aspect, the packaging cover or lidding extends across at least the open portion of the tray to provide a substantially hermetic seal while the package is unopened. In one variation, the cover is a relatively flexible sheet or the like having an adhesive side, at least about its perimeter, which is easily applied to and peeled-away from edges or a lip extending about the open portion of the tray. In another embodiment, the cover is a relatively rigid lid having a substantially planar configuration with a perimeter configured to provide a tight-fit with the open portion of the tray. In particular, the lid may have a contoured perimeter with a shape that conforms to that of the open portion of the tray to provide a snap-fit closure with the tray. In this embodiment, the same material as used for the tray, such as injection molded polymer, may be used to form the lid.

In another embodiment, the packaging may have a clam shell configuration made from either two mating halves or a unitary piece having a hinge, *e.g.,* a living hinge, between two mating portions. The two halves or portions may be similar in configuration, *e.g*., may be mirror images of each other, or may have varying shapes, sizes and/or volumes. The two halves or portions may be relatively rigid and may be field closed by an adhesive about their contacting edges or by a snap-fit mating configuration.

In any embodiment, the packaging may be made of materials which prevent or inhibit air and moisture from entering into an interior of the housing that contains an analyte sensor. Additionally, the packaging, *e.g.,* the tray or one or more of the package housing portions, may include a space or compartment for containing a desiccant material to assist in maintaining an appropriate or desired humidity level within the packaging in order to protect the reagent(s) in the analyte sensor and thereby maintain or extend the sensor's shelf-life and/or desired use-life, *i.e.,* the time period after the sensor is removed from the packaging material, if used. The desiccant may be in a form which minimizes the overall profile of the sensor packaging and minimizes the risk of contamination of the sensor reagent(s) by the desiccant material.

Embodiments of the present disclosure also include methods of packaging analyte sensors, either one by one or collectively in an array format or in a set arrangement, which methods include providing the sensors in the subject packaging. Certain of the methods further include sealing the sensors in a desiccated condition.

Even with nominal variation in sensitivity from sensor-to-sensor upon fabrication of a sensor lot or between sensor lots, factory-calibrated sensors or sensors that do not require any factory calibration may still undergo a drift in sensitivity subsequent to their fabrication due to environmental exposure during the shelf-life of the sensors. To minimize such environmental effects of ambient air, particularly the effects of humidity, to which the sensors may be exposed prior to use, *i.e.,* during their shelf-life, which may be from about 6 to about 18 months or longer, the subject sensors are individually packaged (but may be packaged in pairs or groups) at the factory in removable, sterile packaging which is not to be removed until the enclosed sensor is to be used, *i.e.,* implanted within a user's body.

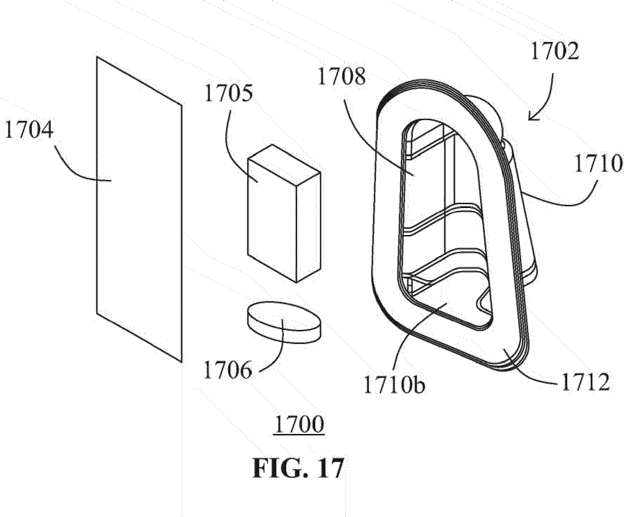

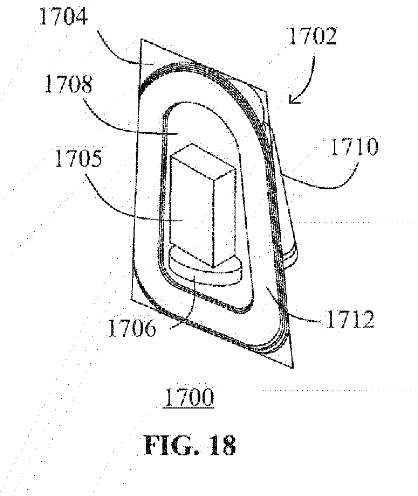

The removable packaging may include one or more housing components and/or materials. In one embodiment, such as that illustrated in FIGS. 17 and 18, the sensor packaging housing 1700 includes a tray 1702 and a lidding or cover 1704 and a desiccant 1706 housed therein. An analyte sensor assembly 1705, including an analyte sensor manufactured in accordance with one or more embodiments described above, which is typically operatively mounted in a sensor inserter with optional safety components (*e.g*., a safety pin) which maintain the sensor within the inserter until released (for example, to initiate sensor insertion), is hermetically sealed within packaging 1700. The present disclosure provides variations of packaging 1700 and its various components in addition to those illustrated and discussed herein. Additional information can be found in US Patent Application No. 12/981,129 entitled "Analyte Sensor and Apparatus for Insertion of the Sensor" filed February 1, 2010.

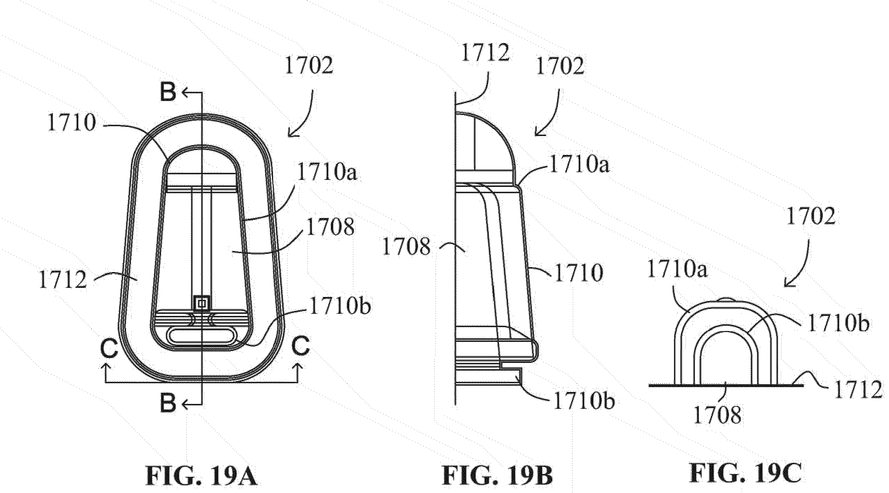

In one variation, as illustrated in FIGS. 19A-19C, tray 1702 has a relatively rigid construct to protect an enclosed sensor assembly 1705 (shown in FIGS. 17 and 18 only) during shipping, handling, and storage over the course of the sensor's shelf-life. The tray 1702 has an open portion or side 1708 through which the sensor 1705 is received and retrieved, and a closed portion or housing 1710 which provides receptacles or compartments 1710a, 1710b within which the sensor assembly 1705 and a desiccant 1706 are held, respectively. The tray housing 1710 may have a shape and size which minimizes the unoccupied volume of the package (i.e., that space which is not occupied by either the sensor assembly 1705 or desiccant 1706) in order to minimize the amount of air within the package 1700. In particular, housing 1710 may be internally contoured to match the shape of the enclosed sensor assembly 1705 and any other packaged contents, *e.g*., desiccant 1706, to further eliminate any excess volume within the enclosed packaging as well as to minimize movement of the sensor assembly 1705 and desiccant 1706 once sealed within the packaging. Tray housing 1710 may be externally contoured to matingly engage or nest within an outer packaging (not shown) or the like. Housing 1710 may be transparent or opaque. Tray 1702 may have an edge or lip 1712 extending radially outward from closed portion 1710 for engaging with a corresponding cover or lidding 1704. Suitable materials for achieving these features and objects for the tray 1702 are injection molded polymers, such as polypropylene.

The packaging cover or lidding 1704 may cover the open portion 1708 of the tray 1702 to provide a substantially hermetic seal while the package 1700 is in an unopened, sealed condition. In one variation, cover 1704 is a relatively flexible sheet or the like having an adhesive side, at least about its perimeter, which is easily applied to and peeled-away from edges or tip 1712 about the open portion 1708 of the tray. Suitable materials for this variation of the cover include aluminum foil, polyethylene film, or the like, or a laminated composite of more than one of these materials. In another variation, the cover may be a relatively rigid lid having a substantially planar configuration with a perimeter configured to provide a tight-fit with the open portion 1708 of the tray. In particular, the lid may have a contoured perimeter (not shown) with a shape that conforms to the inner perimeter of the open portion of the tray to provide a snap-fit closure with the tray. In this variation, the material used to fabricate the tray, such as injection molded polymer, *e.g*., polypropylene, may be used to form the lid.

In another embodiment (not illustrated), the packaging may have at least two relatively rigid components which fit together in a mating fashion. For example, the packaging may have a clam shell configuration interconnected and moveable relatively to each other (for opening and closing) via a hinge, *e.g*., a living hinge. The two halves or portions may be similar in configuration, *e.g*., may be mirror images of each other, or may have varying shapes, sizes and/or volumes. The two halves or portions are preferably relatively rigid and may be held closed by an adhesive about their contacting edges or by a snap-fit mating configuration.

In any embodiment, the analyte sensor packaging may be made of materials which prevent or inhibit moisture and vapor from entering into an interior of the housing that contains an analyte sensor. For example, the moisture and vapor transmission rate (MVTR) of the packaging 1700 of FIGS. 17 and 18, given the necessary dimensions of the tray and lid for a typical-sized sensor/inserter, may be no greater than about 0.5 mg/day, *e.g.,* less than about 0.46 mg/day.

In addition to maintaining a relatively minimal MVTR, the packaging, *e.g.,* the tray 1702 or one or more of the package housing portions, includes a space or compartment 1710b for containing a desiccant material 1706 to assist in maintaining an appropriate humidity level within the packaging in order to protect the reagent(s) in the analyte sensor and thereby maintain or extend the sensor's shelf-life and/or desired use-life, *i.e.,* the time period after the sensor is 1705 removed from the packaging material. The desiccant 1706 may be in a form and have a volume which minimizes the overall profile of the sensor packaging 1700 and minimizes the risk of contamination of the sensor reagent(s) by the desiccant material. In certain embodiments, as illustrated in FIGS. 17 and 18, the desiccant material 1706 is in a unitary solid form, such as a tablet, block or sheet, e.g., in the form of thick paper. In other embodiments (not illustrated), the desiccant may be granular packaged in a sachet or in the form of a gel packet. The unitary piece of desiccant 1706 may be coated with a pharmaceutical grade coating to prevent any shedding of the desiccant material onto the sensor assembly 1705. The mass of the desiccant depends on various factors including, but not limited to the MVTR of the packaging, the packaged component moisture, storage temperature and humidity, etc. The subject desiccants may have an absorption capacity of about 17.5% or greater at typical ambient storage conditions, *i.e.,* about 25°C and about 30%RH, and a safety factor of about 90.0% or greater. Suitable desiccant materials for use with the present invention include, for example, silica gel, calcium sulfate, calcium chloride and molecular sieves. Examples of such desiccants suitable for packaging with a sensor/inserter assembly include, for example, a 2.6 g silica gel tablet and a 10g silica gel pack manufactured by Multisorb Technologies, 325 Harlem Road, Buffalo, New York 14224.

The subject desiccated packaging enables the provision of implantable analyte sensors which are substantially impervious to negative environmental effects from ambient air (at substantially typical storage temperature, humidity and barometric pressure conditions, *i.e.,* at about 25°C, 60%RH and 19.0 mbar) over the course of the sensor's shelf-life (*e.g.,* about 18 months) and use-life (*e.g.,* from about 3 to about 30 days or more, *e.g*., 3 days to about 14 days, *e.g.,* 3 days to about 10 days *e.g.,* 3 days to about 7 days), and may even extend these timeframes. In certain embodiments, the sensor shelf-life may be extended up to about 24 months or more, and the sensor use-life may be extended from about 3 up to about 14 days or more.

Due to the protection provided to the sensors, and particularly to the analyte reagent materials of the sensor, by the subject packaging structures, the sensors' sensitivity is subject to only nominal variations, and thus, may require no user-based calibrations, *i.e.,* the sensors require only factory-calibration. Moreover, in cases where sensor lots are reproducible with sufficiently minimal variation in sensor-to-sensor sensitivity from the outset, no calibration or adjustment of the sensor characteristics during or post manufacturing, nor during *in vivo* use of the sensor may be necessary when packaged with the subject packaging.

The present disclosure also includes methods for packaging implantable analyte sensors for continuous analyte monitoring systems. In one method, the sensor or sensor/inserter assembly is placed in a first packaging component and a second packaging component is sealed to the first packaging component. Sealing may be accomplished by an adhesive or heat-sealing the two components together. With the tray-cover embodiment 1700 of FIGS. 17 and 18, for example, the sensor assembly (sensor and inserter) 1705 is placed in the tray 1702 along with the desiccant 1706, and then cover or lidding 1704 is hermetically sealed to tray 1702 by applying, for example, heat and pressure about the perimeter 1712 of the tray.

### Sensitivity Control With Defined Channel Length

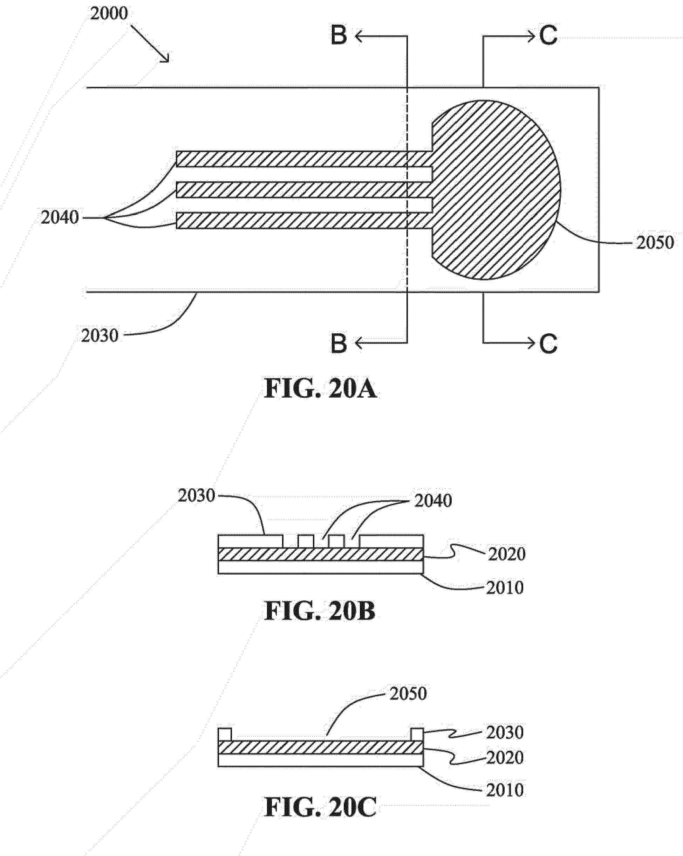

FIG. 20A illustrates a top view of a working electrode of an analyte sensor in one embodiment of the present disclosure, while FIGS. 20B and 20C illustrate cross-sectional views of the working electrode of FIG. 20A at lines B and C, respectively. Referring to FIGS. 20A-20C, the working electrode 2000 may include one or more channels 2040. In certain aspect, channels 2040 are used to define the location and amount of sensing material to be applied to the working electrode 2000. The length L, and number of channels 2040 of the working electrode 2000 may determine the sensitivity of the sensor. In certain embodiments, the channels 2040 are etched into a coverlay material 2030 (see FIG. 20B), which is applied over the conductive layer 2020 of the working electrode 2000. As described, in some embodiments, the conductive layer 2020 may comprise gold, and the conductive layer 2020 of the working electrode 2000 is formed over at least a portion of the length of the substrate 2010 of the sensor.

Referring to FIGS. 20A-20C, in certain embodiments, a well 2050 is etched into the coverlay material 2030 (see FIG. 20C) and is connected to the channels 2040. The well 2050 is used for application of the sensing layer, whereby the sensing layer is deposited into the well 2050 and the sensing layer fills the channels 2040 via capillary action in certain embodiments. After the sensing layer fills the channels 2040 and subsequently dries, the electrode is cut along line B to remove well 2050, leaving only the sensing layer filled channels 2040. In other embodiments, the sensing layer may be deposited directly over the channels 2040 in lieu of using well 2050.

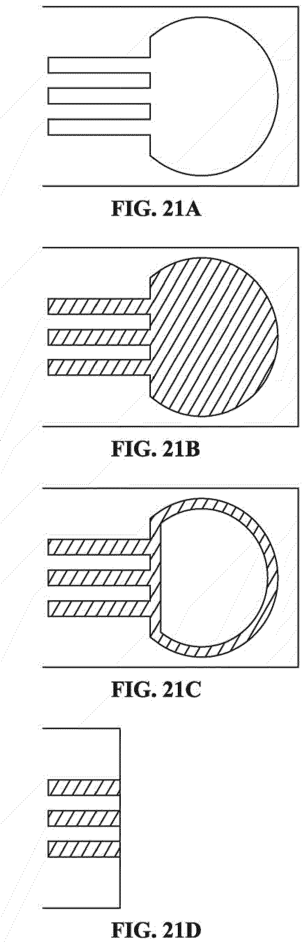

FIGS. 21A-21D illustrate the various stages of sensing layer application to the working electrode of FIG. 20A in one embodiment. Referring now to FIGS. 21A-21D, one or more channels 2040 (FIG. 20A) and a well 2050 are etched into the coverlay material 2030 of a working electrode 2000 (FIG. 21A). The sensing layer is deposited into well 2050 and the channels 2040 are filled with the sensing layer via capillary action, as shown in FIG. 21B. After being deposited, in one embodiment, the sensing layer migrates to the channels 2040 and to the edges of the well 2050, and dries as a ring around the perimeter of the well 2050, as shown in FIG. 21C. The channels 2040 are configured to be narrow in width, such that even as the sensing layer migrates to the edges of the channels 2040, the channels 2040 are narrow enough such that when the sensing layer dries, it still covers substantially all of the conductive area of the channels 2040. As illustrated in FIG. 21D, the working electrode is then cut to remove the well 2050, leaving only the sensing layer filled channels 2040 on the working electrode.

In this manner, in certain aspects of the present disclosure, *in vivo* analyte sensors may include channels for defining conductive substrate (*e.g*., with gold) with sensing layer provided thereon, and techniques for filling the channels and trimming the channels to the desired dimension (such as length) to control the sensor sensitivity (for example, by accurately defining the area of the conductive gold substrate covered by the sensing layer).

### Overall Systems and Algorithms

In a further aspect, programming or executable instructions may be provided or stored in the data processing device of the analyte monitoring system including, for example, the electronics assembly including, for example, data processing unit, memory components, communication components and the like, and/or the receiver/controller unit to provide a time varying adjustment algorithm to the *in vivo* sensors during use. That is, in one embodiment, based on a retrospective statistical analysis of analyte sensors used *in vivo* and the corresponding glucose level feedback, a predetermined or analytical curve or a database may be generated which is time based, and configured to provide additional adjustment to the one or more *in vivo* sensor parameters to compensate for potential sensor drift in stability profile, or other factors.

For example, in the case where the *in vivo* sensor sensitivity decreases for a certain time period measured from the initial sensor insertion or transcutaneous positioning, the sensor sensitivity may approach a steady state level over a given time period (for example, but not limited to, one or two day period from the initial sensor insertion). Accordingly, a database such as for example, a look up table with varying time based adjustment criteria or factors may be provided or programmed in the data processing unit of the electronics assembly and/or the receiver/controller unit such that during a predetermined post-manufacturing time period, *e.g*., the initial about 24 hours to about 36 hours from the initial *in vivo* sensor insertion, the stored adjustment parameter from the look tip table may be applied to modify or otherwise compensate for the expected sensitivity variation during the initial 24 or 36 hour time period (or some other suitable time period as may be statistically determined). In this manner, in certain embodiments, sensor behavior may be statistically estimated during manufacturing, testing, and/or sensor characterization to generate or determine a schedule of sensitivity adjustments for automatic implementation by the CGM system during *in vivo* use of the analyte sensor.

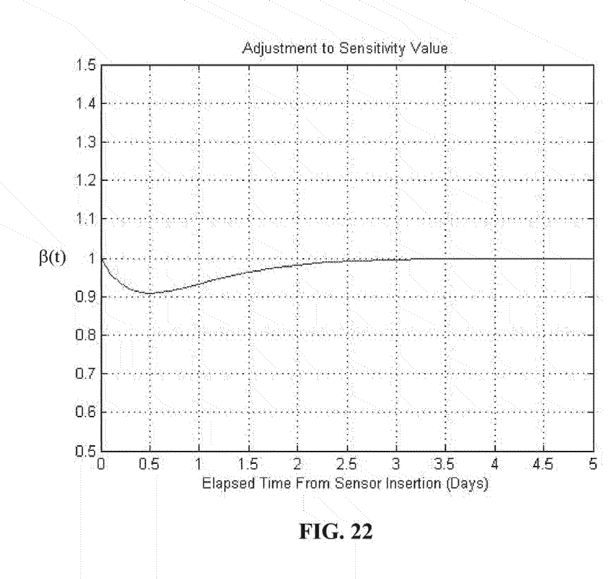

FIG. 22 illustrates an exemplary time varying sensitivity drift profile associated with an analyte sensor for use in the analyte monitoring system in accordance with one embodiment of the present disclosure. As shown in FIG. 22, a time varying parameter β(t) may be defined or determined based on analysis of sensor behavior during *in vivo* use, and a time varying drift profile may be determined as shown in FIG. 22, where the defined time varying parameter β(t) may be coded or programmed with each manufactured sensor, and for example, provided automatically to a data processing unit such as the receiver unit of the analyte monitoring system, for example, to apply the time varying parameter β(t) to the signals obtained from the sensor.

That is, in one aspect, using a sensor drift profile such as for example, that shown in FIG. 22, the analyte monitoring system may be configured to compensate or adjust for the sensor sensitivity based on the sensor drift profile. In certain aspects, the compensation or adjustment to the sensor sensitivity may be programmed in the receiver unit or the controller or data processor of the analyte monitoring system such that the compensation or the adjustment or both may be performed automatically and/or iteratively when sensor data is received from the analyte sensor. In an alternate embodiment, the adjustment or compensation algorithm may be initiated or executed by the user (rather than self initiating or executing) such that the adjustment or the compensation to the analyte sensor sensitivity profile is performed or executed upon user initiation or activation of the corresponding function or routine.

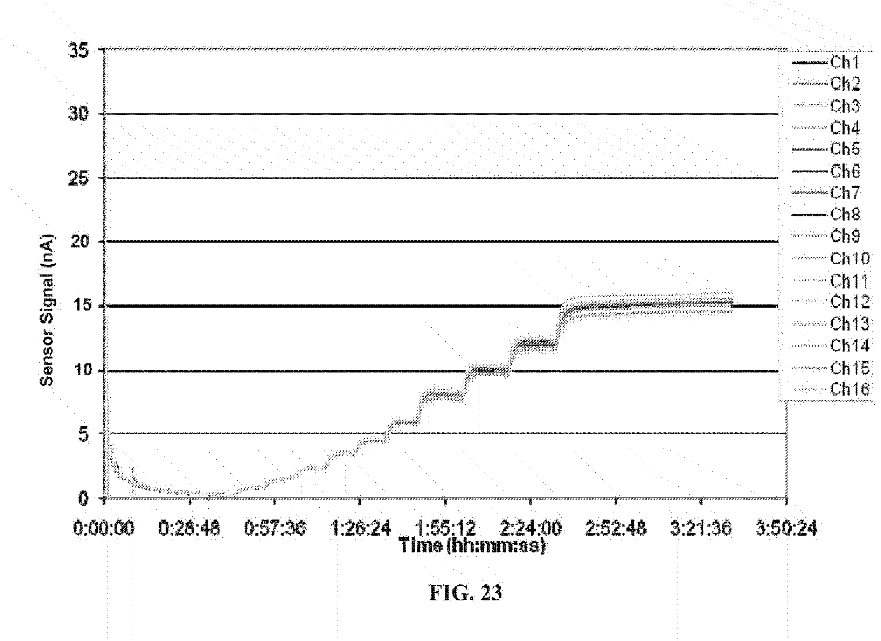

FIG. 23 illustrates sensitivity variation of 16 analyte sensors from a sensor lot manufactured in accordance with the process(es) described above in response to an *in vitro* testing. More specifically, 16 analyte sensors were tested in an *in vitro* testing condition (*e.g*., in a beaker) having a known solution of glucose concentration to determine the sensor response. Referring to FIG. 23, it can be observed that over approximately a four hour time period, each of the 16 sensors exhibited a substantially consistent response or sensitivity to the gradual increase of the glucose concentration. That is, each of the 16 sensors of the same manufactured sensor lot responded in a very similar manner to the same known glucose concentrations. For example, referring back to FIG. 23, each step shown in the plot for each of the 16 sensors is associated with an increase of the glucose concentration (over the time period shown in the X axis) and the sensor response to the increased glucose concentration shown in the Y axis.

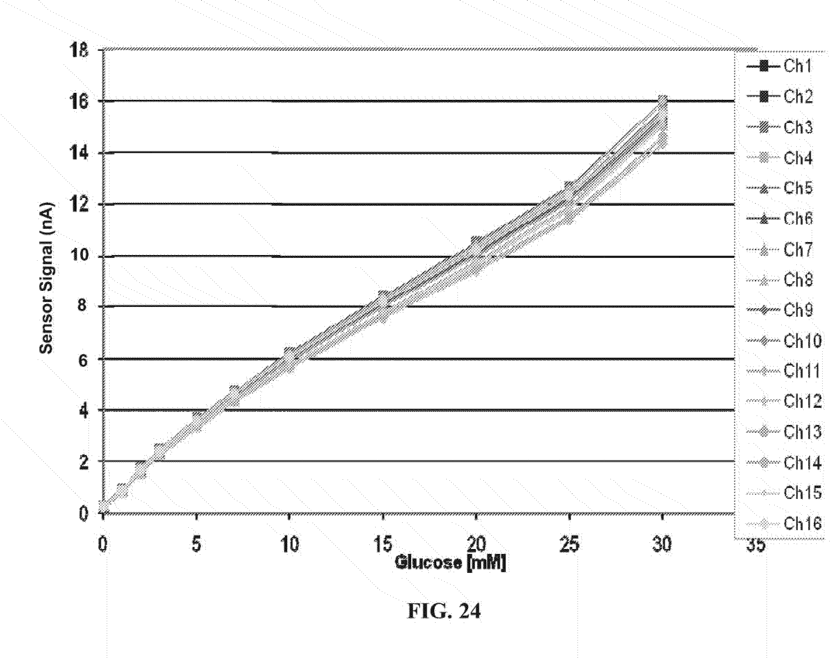

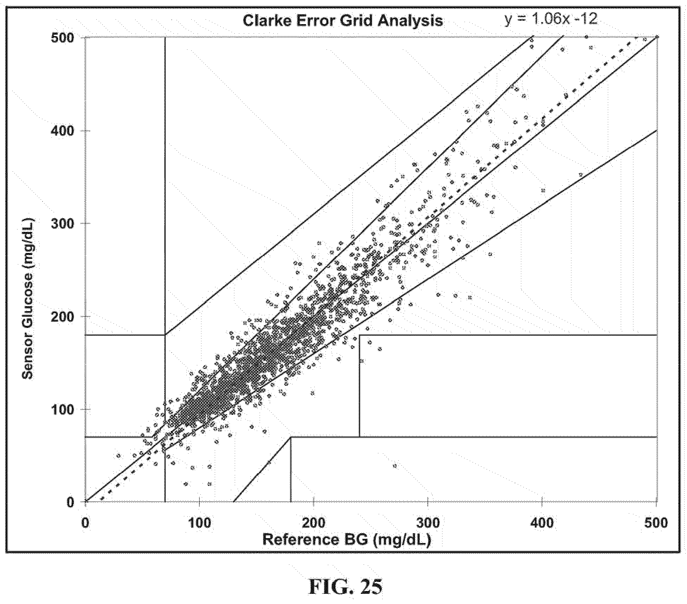

In other words, referring still to FIG. 23, it can be seen that each of the 16 sensors that were tested in a beaker with known glucose concentration exhibited almost identical or very similar response compared to each other (*i.e.,* current signal generated by each sensor) to the glucose concentration in the beaker solution. The results or response of the 16 sensors tested in the beaker solution based on the known glucose concentration level is shown in FIG. 24. That is, referring to FIG. 24, the 16 sensors manufactured in accordance with the process(es) described above, when tested *in vitro* as described above, exhibited the response or characteristics as shown in FIG. 24 where it can be seen that all 16 sensors' signal response to the gradual increase in the glucose concentration in the beaker solution is substantially consistent. That is, it can be observed from the experimental results that the coefficient of variation of the 16 sensors tested *in vitro* is less than approximately 5%, and more specifically, approximately 3%. Sensors from the same manufacturing lot as those 16 sensors tested *in vitro* and the results described above were further tested or used *in vivo* in subjects with diabetic condition, the results of which are described and illustrated below in conjunction with FIG. 25.

FIG. 25 is a Clarke Error Grid based on analyte sensors manufactured in accordance with the one or more embodiments of the present disclosure described above. More particularly, data from 24 sensors manufactured in accordance with the one or more embodiments described above were obtained based on twelve diabetic subjects that wore each sensor for a five day period for two cycles (*e.g.,* for a total of about ten days). It is to be noted that the experimental results set forth herein included simulated factory calibration by applying one calibration factor or parameter to each of the 24 sensors, where the calibration factor was retrospectively determined.

The resulting data from the 24 sensors are additionally shown below in the table that illustrates 87.4% of data points obtained that are in the Zone A (clinically accurate) of the Clarke Error grid, while 11.9% of the data points obtained are in the Zone B (clinically acceptable) of the Clarke Error grid.

| EGA Statistics | | | | | | | |
|---|---|---|---|---|---|---|---|
| | A | B | C | D-La | D-Hi | E-Lo | E-Ni |
| Points | 1464 | 200 | 1 | 7 | 3 | 0 | 1 |
| % | 87.4% | 11.9% | 0.1% | 0.4% | 0.2% | 0.0% | 0.1% |
| % A+B | 99,3% | | | | | Total | 1676 |
| | | | | | | | |

| Orthogonal Reg | | Range | N | MRD | MARD | | |
|---|---|---|---|---|---|---|---|
| Slope | 1.06 | All | 1676 | -0.5% | 10.6% | | |
| Intercept | -12.0 | [0,100) | 188 | 7.2% | 14.7% | | |
| R2 | 094 | [100,180] | 874 | -1.3% | 10.4% | | |
| RMSE | 16.7 | [180,240) | 389 | -2.3% | 9.1% | | |
| MARD 90pct | 22.4% | [240 - ] | 225 | -0.6% | 10.3% | | |

Using a single calibration factor for all sensors in the sensor lot provides accuracy of approximately 99.3% in the combined Zones A and B of the Clark Error grid. Based on the foregoing and results described above of the sensors from the manufacturing lot of sensors, the results obtained from the beaker testing to determine sensor response and the *in vivo* sensor response in diabetic subjects exhibit very similar characteristics, resulting in predictable sensor sensitivity, such that the results of factory calibration is clinically acceptable sensor accuracy. Accordingly, it can be seen that the sensors manufactured in accordance with the embodiments described above provide minimal or insubstantial sensitivity variation such that user initiated calibration of the sensor during *in vivo* use in certain embodiments is obviated.

Embodiments also include determination of a normalization curve or slope (or a definable functional relationship) based on a select number of sample sensors within a manufacturing sensor lot, such as for example, 10 sample sensors from a sensor lot of 1,000 sensors or more, or 16 sample sensors from a sensor lot of 1,000 sensors or more, or 25 sample sensors from a sensor lot of 1,000 sensors or more, etc. With the defined sample size of the sensor lot, the characteristics or parameters of each of the sample sensors from the sensor lot are determined including, for example, the membrane thickness at one or multiple points, or the size of the active sensing area including, for example, the surface area, volume, height, length, and/or shape (such as concave, convex, flat or sloped, for example, measured at one or multiple points) of the active area defined on the sensor. Thereafter, in certain embodiments, a mean value of these characteristics may be determined by for example, averaging the measured values to determine, for example, the mean membrane thickness of the sample sensors of the sensor lot, the mean membrane thickness at one or more points on the membrane surface, the mean surface area of the sensing area or the mean dimensions of the sensing area and/or the mean surface area thickness at one or more points on the active area surface. In addition, in certain embodiments, coefficient of variation (CV) of these measured or determined parameters or characteristics from the sample sensors of the sensor lot is determined. In addition, the sensitivity of each of the sample sensors of the sensor lot may be determined.

Based on the determination of the sample sensor characteristics described above, embodiments include comparison of the determined characteristics to an accepted value or level of each determined value or characteristics of the sample sensors to determine whether the sample sensors exhibit characteristics that are within the acceptable criteria or range. For example, mean values for the sensitivity of the sample sensors may be compared against a predetermined sensitivity that is correlated with a sensor sensitivity having coefficient of variation of less than 5%, or less than 3%, or the like. If the comparison results in an acceptable mean sensitivity value, then the entire sensor lot is accepted and the mean sensitivity value determined based on the sample sensors are assigned to each sensor in the sensor lot.

According to the invention, each sensor in the sensor lot (other than those sample sensors) is examined non-destructively to determine or measure its membrane thickness at one or more points of the sensor, and optionally other characteristics including physical characteristics such as the surface area/volume of the active area may be measured or determined. Such measurement or determination may be performed in an automated manner using, for example, optical scanners or other suitable measurement devices or systems, and the determined sensor characteristics for each sensor in the sensor lot is compared to the corresponding mean values based on the sample sensors for possible correction of the calibration parameter or code assigned to each sensor. For example, for a calibration parameter defined as the sensor sensitivity, the sensitivity is approximately inversely proportional to the membrane thickness, such that, for example, a sensor having a measured membrane thickness of approximately 4% greater than the mean membrane thickness for the sampled sensors from the same sensor lot as the sensor, the sensitivity assigned to that sensor in one embodiment is the mean sensitivity determined from the sampled sensors divided by 1.04. Likewise, since the sensitivity is approximately proportional to active area of the sensor, a sensor having measured active area of approximately 3% lower than the mean active area for the sampled sensors from the same sensor lot, the sensitivity assigned to that sensor is the mean sensitivity multiplied by 0.97. The assigned sensitivity may be determined from the mean sensitivity from the sampled sensors, by multiple successive adjustments for each examination or measurement of the sensor. In certain embodiments, examination or measurement of each sensor may additionally include measurement of membrane consistency or texture in addition to the membrane thickness and/or surface are or volume of the active sensing area.

In certain embodiments, each sensor of the sensor lot may be independently analyzed or examined using, for example, optical or other suitable measurement devices or systems, to determine its characteristics, such as, for example, but not limited to, the membrane thickness at one or more locations of the sensor, consistency and/or texture of the membrane, the size, surface area, volume, and/or dimension of the active area including, for example, the geometry of the active area may be measured optically or otherwise, and each of the measured parameters for each sensor is compared to a predetermined value or range of values stored in a database or a storage medium, where the predetermined value or range of values correspond to values or range of values that are considered to be acceptable such that when the measured sensor values correspond to the predetermined value or range of values, the sensor characteristics is considered to be within an acceptable coefficient of variation (CV), for example, within about 5%, within about 3% or less, or within about 1% or less. The sensitivity that is assigned to the particular sensor may be determined in this manner without determining the sensitivity with lot sampling (that is, for example, sampling each sensor in the sensor lot to determine the sensitivity). Alternatively, the sensitivity determined from these measurements may be confirmed with a sensor lot sample mean sensitivity, for example, as part of a verification procedure during manufacturing.

Embodiments further include the time varying drift profile programmed or programmable in the receiver unit or the transmitter unit of the CGM system as a database or a look up table or otherwise stored in a memory unit or storage device, and constructed with a suitable adjustment or modification value for each hour time period measured from the initial sensor insertion, and thereafter, starting from the initial *in vivo* use of the sensor, the corresponding value in the look up table is retrieved and applied or otherwise factored into the sensor sensitivity such that the sensor output data is representative of the monitored glucose level.

In certain embodiment, a calibration parameter or code is loaded or programmed into the memory unit or the data processing unit of the electronics assembly physically coupled with the analyte sensor. The programming or loading of the calibration parameter or code may be accomplished by a serial command, for example, using a wired or wireless connection to one or more communication ports of the electronics assembly. During *in vivo* use, in one embodiment, a receiver/controller unit is configured to query the electronics assembly and retrieve the calibration parameter or code loaded or programmed in the memory or storage device of the electronics assembly for use in converting the measured raw sensor signals from the analyte sensor to the corresponding glucose values. Alternatively, the sensor electronics may include programming to perform this conversion.

In certain embodiments where sensors exhibit drift (*e.g*., where the sensor sensitivity drifts an expected percentage over a certain time), a drift profile may be defined by an algorithm of the monitoring system to determine a drift correction factor that may be applied to sensor signal to obtain a glucose measurement (mg/dL). Due at least in part to the high reproducibility of the manufacturing process that results in low manufacturing coefficient of variation (CV), a single drift correction factor may be used for all sensors of a given sensor manufacturing lot or batch.

Accordingly, because the sensitivity of each sensor of a given manufacturing lot are substantially the same according to the embodiments herein, the factory-determined sensitivity or calibration parameter may be applied to all sensors of such a lot, *i.e.,* a single calibration algorithm may be used for all the sensors of a given lot. According to the invention, this calibration code or parameter is programmed or is programmable into software of the monitoring system, *e.g*., into one or more processors. According to the invention, the factory-determined calibration parameter or code may be provided to a user with a sensor(s) and uploaded to a calibration algorithm manually or automatically (*e.g*., via bar code and reader, or the like), or pre-stored in the memory or storage device of the analyte monitoring system. Calibration of the sensor signal is then be implemented using suitable hardware/software of the system.

In the manner described, in accordance with various embodiments of the present disclosure, a continuous analyte monitoring system with analyte sensors manufactured in the manner described above is provided that does not require user performed sensor calibration during *in vivo* use. In certain aspects, the analyte sensors are highly reproducible with at least negligible sensor to sensor variation, and which exhibit substantially stable sensor profiles post manufacturing and prior to positioning in a user.

Additionally, embodiments of the analyte sensors of the present disclosure include predictable sensitivity drift determined during *in vivo* use to minimize potential *in vivo* variation whereby one or more defined algorithms programmed or programmable (either during manufacturing or programmed during use) in the data processing unit or the receiver unit of the CGM system for a given sensor drift profile are applied for a correction or adjustment to the CGM system to eliminate the need for user calibration. Such correction or adjustment to the CGM system may include one or more feedback algorithm programmed or programmable in the analyte monitoring system to apply a correction or adjustment profile or template determined *a priori,* or in real time by the CGM system such that adjustment to the sensor stability profile and thus the accuracy of the reported glucose values from the sensors during *in vivo* use is maintained within clinically acceptable range. In this manner, in certain aspects of the present disclosure, any clinically significant person to person variation in the ratio of interstitial to blood glucose concentration ascertained during *in vivo* sensor use may be compensated by one or more feedback algorithm or routines programmed in the CGM system. In one aspect, the one or more feedback algorithm or routines may include the *in vivo* sensor response collected, analyzed and profiled for each particular subject or the user such that the analyzed and profiled information associated with the particular user of the analyte monitoring system may be stored in a memory or storage device of the analyte monitoring system or elsewhere and used or applied to the signals from the *in vivo* sensor during use.

Accordingly, in certain embodiments, *in vivo* sensors that do not require user or system based calibration may be provided by minimizing variation in sensor characteristics during or post manufacturing by providing, for example, defined, and reproducible active area of the sensor, controlling the sensor membrane thickness and enzyme stability, and further, providing a substantially stable post manufacturing environment to maintain stable sensor profile during its shelf life by controlling the relative humidity, and packaging configuration, for example, to provide storage conditions that are substantially impervious to negative environmental effects post manufacturing, and prior to *in vivo* use.

In one embodiment, an analyte sensor may comprise a substrate, a conductive layer disposed over at least a portion of the substrate, and a sensing layer disposed substantially orthogonally over at least a distal portion of the conductive layer wherein the area of the sensing layer as at least as large as the area of the distal portion of the conductive layer.

The distal portion of the conductive layer may have a width greater than that of a proximal portion of the conductive layer.

The distal portion of the conductive layer may terminate proximally of a distal edge of the substrate.

In another embodiment, a method of fabricating an analyte sensor may comprise disposing the sensing layer over the entirety of the distal portion of the conductive layer.

In yet another embodiment, an analyte sensor may comprise a substrate, a conductive layer disposed over at least a portion of the substrate, and a sensing layer disposed substantially orthogonally over at least portion of the conductive layer wherein the width of the sensing layer is substantially continuous.

The sensing layer may comprise a strip or band of sensing material.

The conductive layer may extend to a distal edge of the substrate.

The conductive layer may terminate proximally of a distal edge of the substrate.

In one aspect, there may be substantially no sensor-to-sensor sensitivity variation.

In another embodiment, a method of fabricating an analyte sensor may comprise disposing the sensing layer in a strip having a substantially constant width.

In yet another embodiment, a method of fabricating a plurality of analyte sensors may comprise providing a substrate, disposing a conductive layer over the substrate, wherein the conductive layer forms a plurality of electrodes, disposing the sensing layer in a strip having a substantially constant width over the plurality of electrodes, wherein the strip is substantially orthogonal to each of the plurality of electrodes, and singulating the substrate into a plurality of sensors.

In yet another embodiment, an analyte sensor may comprise a substrate, a conductive layer disposed over at least a portion of the substrate, a dielectric layer disposed over the conductive layer and having a void or well therein, and a sensing layer disposed within the void.

The void may be located over a distal portion of the conductive layer.

Embodiments include the void or well having a varying dimension within the defined active area and/or along the distal portion of the sensor. By way of non-limiting exemplary illustrations, the void or well may be shaped substantially circular with a gradually varying depth towards the center of the circular shape such that the center of the circular shape is deeper compared to the circumference portion of the void, the depth may be substantially constant, or gradually varying away from the center of the circular shape such that the circumference portion of the void or well is relatively deeper compared to the center of the circular shape.

Embodiments also include the void or well having a circular, rectangular, triangular or other geometry as may be suitable. Each such geometry may further include variations in one or more dimensions including volume, surface area, height of the void or well, and depending upon the geometry, diameter or length of the void.

In another embodiment, a method of fabricating an analyte sensor may comprise providing a substrate, disposing a conductive layer over the substrate, disposing a dielectric layer over the conductive layer, wherein the dielectric layer has a void therein, and disposing a sensing material within the void.

In yet another embodiment, an analyte sensor may comprise a substrate comprising an implantable portion having a length and a width, a first conductive trace disposed over the entire length and width of a first side of the substrate, a second conductive trace disposed over the entire length and width of a second side of the substrate, and a sensing material in the form of a stripe disposed over at least a portion of the first conductive trace defining an active area, wherein the stripe of sensing material is substantially orthogonal to the length of the substrate.

The substrate may further comprise a non-implantable portion and the sensor further comprises a third conductive trace disposed over at least a portion of the non-implantable portion.

The first conductive trace may function as a working electrode and the second conductive trace functions at least as a reference electrode.

The third conductive trace may function as a counter electrode.

Furthermore, at least one membrane may be disposed over the sensing material.

A first membrane may modulate the flux of analyte to the sensing material.

The first membrane may be disposed over the sensing material in the form of a stripe positioned substantially orthogonally to the length of the implantable portion of the substrate.

A second membrane may provide a conformal coating over at least the implantable portion of the substrate.

The second conductive trace may comprise a primary layer covering the entire surface area of the second side of the implantable portion of the substrate and a secondary layer in the form of a stripe disposed over at least a portion of the primary layer wherein the secondary layer is substantially orthogonal to the length of the substrate.

The substrate width may be in the range from about 0.05 mm to about 0.6 mm, and wherein the width of the sensing material is in the range from about 0.05 mm to about 5 mm.

The active area may be in the range from about 0.0025 mm² to about 3 mm².

Furthermore, a dielectric layer may be disposed over at least a portion of the first conductive trace but not disposed over at least a top surface of the sensing material.

The dielectric layer may be provided in two spaced-apart portions and the sensing material is disposed between the spaced-apart portions.

In another embodiment, a method of fabricating an analyte sensor to having an active area defined by an overlapping area of a conductive layer and a sensing layer, wherein the active area has a desired surface area may comprise disposing a conductive material on a surface of a substrate to form a conductive layer, disposing a sensing material over at least a portion of the conductive layer to form a sensing layer, and removing a portion of at least the sensing layer to provide a desired surface area of an active area, wherein an overlapping area of the conductive layer and the sensing layer is at least as great as the desired surface area of the active area.

The sensing layer may overlap the conductive layer at a distal portion of the conductive layer.

The surface area of the sensing layer may be greater than the surface area of the distal portion of the conductive layer prior to removing the portion of at least the sensing layer.

The surface area of the distal portion of the conductive layer may be greater than the surface area of the sensing layer prior to removing the portion of at least the sensing layer.

The surface area of the sensing layer and the surface area of the distal portion of the conductive layer may be substantially equal prior to removing the portion of at least the sensing layer.

The surface area of the sensing layer and the surface area of the distal portion of the conductive layer may be different from each other after removing the portion of at least the sensing layer.

The surface area of the sensing layer and the surface area of the distal portion of the conductive layer may be substantially the same after removing the portion of at least the sensing layer.

The shape of the sensing layer and the shape of the distal portion of the conductive layer may be substantially the same after removing the portion of at least the sensing layer.

Only the portion of the sensing layer may be removed.

The portion of the sensing layer removed may circumscribe an edge of a distal portion of the conductive layer.

Furthermore, the method may include removing a portion of the conductive layer to provide the desired surface area of the active area.

The portion of the conductive layer removed may circumscribe an edge of the sensing layer.

The portion of the sensing layer removed and the portion of the conductive layer removed may overlap.

The portion of the sensing layer and the portion of the conductive layer may be removed simultaneously.

No calibration of the analyte sensor may be performed upon fabrication of the sensor.

The step of removing may comprise laser trimming.

A pulsed output of the laser employed may comprise a wavelength in the range of ultraviolet light.

The wavelength may comprise a range from about 266 nm to about 355 nm.

The laser employed may be an ultrafast laser.

The laser employed may be a diode pumped solid state laser.

The laser employed may be a fiber laser.

**In** one aspect, a plurality of analyte sensors fabricated may include substantially no sensor-to-sensor sensitivity variation.

In another embodiment, a method of providing an implantable analyte sensor for use with a continuous analyte monitoring system may comprise performing a batch calibration for the sensor, and packaging the batch calibrated sensor within a hermetically sealed housing containing a desiccant, wherein the housing has a relatively low moisture and vapor transmission rate.

Furthermore, the method may include desiccating the packaged sensor, wherein the coefficient of variation in sensor sensitivity within the sensor batch is no greater than 10%.

The coefficient of variation in sensor sensitivity within the sensor batch may be no greater than 5% in-vitro.

The coefficient of variation in sensor sensitivity within the sensor batch may be no greater than 10% in-vivo.

Furthermore, the method may include storing the packaged sensor wherein the conditions inside the package in which the sensor is stored comprise about 30%RH, wherein the desiccant has an absorption capacity of at least about 17%.

The ambient conditions in which the packaged sensor is stored may comprise about 25°C and about 30%RH, wherein the desiccant has a safety factor of at least about 90.0%.

Embodiments include sensors with a predictable shelf-life sensitivity drift.

Embodiments include sensors with substantially no shelf-life sensitivity drift.

Embodiments include sensors with a predictable *in vivo* sensitivity drift.

Embodiments include sensors with substantially no *in vivo* drift.

Embodiments include sensor packaging comprising compartmentalizing the desiccant from the sensor.

In another embodiment, a method of providing implantable analyte sensors from the same manufacturing lot for use with a continuous analyte monitoring system may comprise calibrating the sensor batch wherein the coefficient of variation in sensitivity amongst the sensors is no greater than about 5%, and individually packaging the batch calibrated sensors, each within a hermetically sealed housing containing a desiccant, wherein the housing has a relatively low moisture and vapor transmission rate.

Embodiments include storing the packaged sensors wherein the ambient conditions in which the packaged sensors are stored may comprise about 25°C and about 30%RH, wherein the desiccant may have an absorption capacity of at least about 17.5%.

The ambient conditions in which the packaged sensor is stored may comprise about 25 °C and about 30%RH, wherein the desiccant has a safety factor of at least about 90.0%.

Embodiments include an analyte sensor comprising a substrate, a conductive layer disposed over at least a portion of the substrate, a dielectric layer disposed over the conductive layer and having a void therein, and a sensing layer disposed within the void, wherein the area of the sensing layer in contact with the conductive layer has a sensor-to-sensor coefficient of variation of less than approximately 5% within a sensor lot.

Embodiments include the coefficient of variation less than approximately 3% within the sensor lot.

Embodiments further include a membrane disposed over the area of the sensing layer in contact with the conductive layer, wherein the membrane has a defined thickness with a sensor to sensor coefficient of variation of less than approximately 5% within the sensor lot.

Embodiments include the membrane disposed over the area of the sensing layer in contact with the conductive layer having a substantially uniform thickness.

Embodiments include the membrane disposed over the area of the sensing layer in contact with the conductive layer having a substantially uniform distribution.

Embodiments include the membrane having a low oxygen permeability.

Embodiments include the area of the sensing layer in contact with the conductive layer substantially defining an active area of the sensor.

Embodiments include the void being located over a distal portion of the conductive layer.

Embodiments include the conductive layer in contact with the sensing layer defining at least a portion of a working electrode of the analyte sensor.

Embodiments include the conductive layer including one or more of vitreous carbon, graphite, silver, silver-chloride, platinum, palladium, platinum-iridium, titanium, gold or, iridium.

Embodiments include the dielectric layer including a photo-imageable polymeric material.

Embodiments include the dielectric layer including a photo-imageable film disposed over the conductive layer and at least a portion of the substrate.

Embodiments include the void being formed by a photolithographic process.

Embodiments further include one or more of a glucose flux limiting layer, an interference layer or a biocompatible layer disposed over the void.

Embodiments include the area of the sensing layer in contact with the conductive layer being about 0.01 mm² to about 1.0 mm².

Embodiments include the area of the sensing layer in contact with the conductive layer being about 0.04 mm² to about 0.36 mm².

Embodiments include the surface area of the sensing layer in contact with the conductive layer on the substrate being substantially fixed.

Embodiments include the dimension of the void formed in the dielectric layer being substantially fixed.

In another embodiment, an analyte sensor comprises a substrate having a distal portion, a conductive layer disposed over at least a portion of the distal portion of the substrate, a dielectric layer disposed over the conductive layer and having a void therein such that the location of the void coincides with the distal portion of the substrate, and a sensing layer disposed within the void, wherein the area of the sensing layer in contact with the conductive layer has a sensor-to-sensor coefficient of variation of less than approximately 5% within a sensor lot, wherein the distal portion of the substrate is maintained in fluid contact with an interstitial fluid over a predetermined time period.

Embodiments include the predetermined time period being about three days or more.

Embodiments include the area of the sensing layer in contact with the conductive layer defining at least a portion of a working electrode of the analyte sensor in fluid contact with the interstitial fluid over the predetermined time period.

Embodiments include the analyte sensor further including a membrane disposed over the area of the sensing layer in contact with the conductive layer, wherein the membrane has a defined thickness with a sensor to sensor coefficient of variation of less than approximately 5% within the sensor lot.

Embodiments include the membrane disposed over the area of the sensing layer in contact with the conductive layer having a substantially uniform thickness.

Embodiments include the membrane disposed over the area of the sensing layer in contact with the conductive layer having a substantially uniform distribution.

Embodiments include the surface area of the sensing layer in contact with the conductive layer on the substrate being substantially constant between sensors in the sensor lot.

Embodiments include the dimension of the void formed in the dielectric layer being substantially constant between sensors in the sensor lot.

Embodiments further include one or more of a glucose flux limiting layer, an interference layer or a biocompatible layer disposed over the void.

Various other modifications and alterations in the structure and method of operation of the embodiments of the present disclosure will be apparent to those skilled in the art without departing from the scope of the present disclosure. Although the present disclosure has been described in connection with certain embodiments, it should be understood that the present disclosure as claimed should not be unduly limited to such embodiments. It is intended that the following claims define the scope of the present invention.

## Claims

1. A method of manufacturing a batch of in vivo analyte sensor systems, comprising:
forming multiple in vivo analyte sensor systems, each in vivo analyte sensor system comprising:
an in vivo analyte sensor (800) comprising:
a substrate (802);
a conductive layer (804, 804a, 804b) disposed over at least a portion of the substrate;
a dielectric layer (808) disposed over the conductive layer and having a void (810) therein;
a sensing layer (806) disposed within the void and formed over the conductive layer to define an active area of the analyte sensor that generates one or more signals corresponding to a monitored analyte level after insertion of the analyte sensor and when maintained in fluid contact with interstitial fluid, wherein the area of the sensing layer in contact with the conducting layer has a sensor-to-sensor coefficient of variation of less than approximately 5% within the batch; and
a membrane layer formed on the sensing layer, wherein the membrane layer has a defined thickness with a sensor-to-sensor coefficient of variation of less than approximately 5% within the batch; and
a sensor electronics assembly comprising a data processor,
determining the thickness of the membrane layer of each analyte sensor in the batch and at one or more points of the membrane layer;
determining a calibration parameter based on a mean of the determined thicknesses of the membrane layers of the batch of analyte sensors; and
storing the determined calibration parameter in the data processor of the sensor electronics assembly of each of the in vivo analyte sensor systems in the batch, or providing the determined calibration parameter with each of the in vivo analyte sensor systems in the batch, during manufacture of the analyte sensor systems;
wherein the data processor is configured to correlate the generated one or more signals with the monitored analyte level by applying the determined calibration parameter to the generated one or more signals.

2. The method of manufacturing a batch of in vivo analyte sensor systems of any preceding claim, comprising forming an in vivo analyte sensor by:
providing the substrate;
disposing the conductive layer over at least a portion of the substrate;
disposing the dielectric layer over the conductive layer;
forming the void in the dielectric layer;
disposing the sensing layer within the void and on the conductive layer to define an active area of the analyte sensor that generates one or more signals corresponding to a monitored analyte level after insertion of the analyte sensor and when maintained in fluid contact with interstitial fluid; and
forming the membrane layer on the sensing layer.

3. The method of forming an in vivo analyte sensor of claim 2, wherein the step of forming a void in the dielectric layer comprises forming the void with a dimensional range of about 0.01mm to about 1.0 mm optionally of about 0.04 mm² to about 0.36 mm².

4. The method of forming an in vivo analyte sensor of claim 2 or 3, wherein the step of forming a void in the dielectric layer comprises forming the void with a height of the void being gradually increased or decreased and/or having a surface area shaped such that it is tapered, or otherwise varied.

5. The method of forming an in vivo analyte sensor of claim 2, 3 or 4, wherein the step of forming a void in the dielectric layer comprises forming the void using photolithographic techniques, optionally comprising photo-etching to a depth sufficient to expose the conductive layer.

6. The method of manufacturing a batch of in vivo analyte sensor systems of any preceding claim, further comprising: loading the determined calibration parameter of at least one of the in vivo analyte sensor systems in the batch into a memory associated with that analyte sensor system.

7. The method of forming an in vivo analyte sensor of any one of claims 2 to 6, wherein the dielectric layer is formed from a photo-imageable polymeric material or a photo-imageable film disposed over the conductive layer and at least a portion of the substrate.

8. The method of forming an in vivo analyte sensor of any one of claims 2 to 7, wherein the sensing layer is formed with an area of about 0.01 mm² to about 1.0 mm², optionally about 0.04 mm² to about 0.36 mm².

9. The method of manufacturing a batch of in vivo analyte sensor systems of any preceding claim, wherein the sensing layer is formed on the conductive layer.

10. The method of manufacturing a batch of in vivo analyte sensor systems of any preceding claim, wherein the sensor electronics assembly is coupleable with the analyte sensor.

## Patentansprüche

1. Verfahren zur Herstellung einer Charge von In-vivo-Analytsensorsystemen, das Folgendes beinhaltet:
Bilden mehrerer In-vivo-Analytsensorsysteme, wobei jedes In-vivo-Analytsensorsystem Folgendes umfasst:
einen In-vivo-Analytsensor (800), der Folgendes umfasst:
ein Substrat (802);
eine leitende Schicht (804, 804a, 804b), die über mindestens einem Teil des Substrats angeordnet ist;
eine dielektrische Schicht (808), die über der leitenden Schicht angeordnet ist und einen Hohlraum (810) darin aufweist;
eine Sensorschicht (806), die innerhalb des Hohlraums angeordnet und über der leitenden Schicht ausgebildet ist, um einen aktiven Bereich des Analytsensors zu definieren, der ein oder mehrere Signale entsprechend einem überwachten Analytpegel erzeugt, nachdem der Analytsensor eingesetzt wurde und wenn er in Fluidkontakt mit interstitiellem Fluid gehalten wird, wobei der Bereich der Sensorschicht, der mit der leitenden Schicht in Kontakt ist, einen Sensor-zu-Sensor-Variationskoeffizienten von weniger als etwa 5% innerhalb der Charge aufweist; und
eine auf der Sensorschicht ausgebildete Membranschicht, wobei die Membranschicht eine definierte Dicke mit einem Sensor-zu-Sensor-Variationskoeffizienten von weniger als etwa 5 % innerhalb der Charge aufweist; und
eine Sensorelektronikbaugruppe, die einen Datenprozessor umfasst,
Bestimmen der Dicke der Membranschicht jedes Analytsensors in der Charge und an einem oder mehreren Punkten der Membranschicht;
Bestimmen eines Kalibrierungsparameters auf der Basis eines Mittelwertes der bestimmten Dicken der Membranschichten der Charge von Analytsensoren; und
Speichern des bestimmten Kalibrierungsparameters in dem Datenprozessor der Sensorelektronikbaugruppe jedes der In-vivo-Analytsensorsysteme in der Charge oder Bereitstellen des bestimmten Kalibrierungsparameters mit jedem der In-vivo-Analytsensorsysteme in der Charge während der Herstellung der Analytsensorsysteme;
wobei der Datenprozessor zum Korrelieren der erzeugten ein oder mehreren Signale mit dem überwachten Analytpegel durch Anwenden des bestimmten Kalibrierungsparameters auf die erzeugten ein oder mehreren Signale konfiguriert ist.

2. Verfahren zur Herstellung einer Charge von In-vivo-Analytsensorsystemen nach einem vorherigen Anspruch, das die Bildung eines In-vivo-Analytsensors beinhaltet durch:
Bereitstellen des Substrats;
Anordnen der leitenden Schicht über mindestens einem Teil des Substrats;
Anordnen der dielektrischen Schicht über der leitenden Schicht;
Ausbilden des Hohlraums in der dielektrischen Schicht;
Anordnen der Sensorschicht innerhalb des Hohlraums und auf der leitenden Schicht, um einen aktiven Bereich des Analytsensors zu definieren, der ein oder mehrere Signale entsprechend einem überwachten Analytpegel erzeugt, nachdem der Analytsensor eingeführt wurde und wenn er in Fluidkontakt mit interstitiellem Fluid gehalten wird; und
Ausbilden der Membranschicht auf der Sensorschicht.

3. Verfahren zum Bilden eines In-vivo-Analytsensors nach Anspruch 2, wobei der Schritt des Bildens eines Hohlraums in der dielektrischen Schicht das Bilden des Hohlraums mit einem Abmessungsbereich von etwa 0,01 mm² bis etwa 1,0 mm², optional von etwa 0,04 mm² bis etwa 0,36 mm², beinhaltet.

4. Verfahren zum Bilden eines In-vivo-Analytsensors nach Anspruch 2 oder 3, wobei der Schritt des Bildens eines Hohlraums in der dielektrischen Schicht das Bilden des Hohlraums mit einer allmählich zunehmenden oder abnehmenden Höhe und/oder einem Oberflächenbereich des Hohlraums beinhaltet, der so geformt ist, dass er sich verjüngt oder anderweitig variiert.

5. Verfahren zur Herstellung eines In-vivo-Analytsensors nach Anspruch 2, 3 oder 4, wobei der Schritt des Bildens eines Hohlraums in der dielektrischen Schicht das Bilden des Hohlraums mit photolithographischen Techniken beinhaltet, optional mit Photoätzen bis zu einer Tiefe, die ausreicht, um die leitende Schicht zu exponieren.

6. Verfahren zur Herstellung einer Charge von In-vivo-Analytsensorsystemen nach einem vorherigen Anspruch, das ferner Folgendes beinhaltet: Laden des bestimmten Kalibrierungsparameters von mindestens einem der In-vivo-Analytsensorsysteme in der Charge in einen mit diesem Analytsensorsystem assoziierten Speicher.

7. Verfahren zur Herstellung eines In-vivo-Analytsensors nach einem der Ansprüche 2 bis 6, wobei die dielektrische Schicht aus einem fotoabbildbaren polymeren Material oder einem fotoabbildbaren Film gebildet wird, der über der leitenden Schicht und mindestens einem Teil des Substrats angeordnet ist.

8. Verfahren zur Herstellung eines In-vivo-Analytsensors nach einem der Ansprüche 2 bis 7, wobei die Sensorschicht mit einer Fläche von etwa 0,01 mm² bis etwa 1,0 mm², optional etwa 0,04 mm² bis etwa 0,36 mm², ausgebildet wird.

9. Verfahren zur Herstellung einer Charge von In-vivo-Analytsensorsystemen nach einem vorherigen Anspruch, wobei die Sensorschicht auf der leitenden Schicht ausgebildet ist.

10. Verfahren zur Herstellung einer Charge von In-vivo-Analytsensorsystemen nach einem vorherigen Anspruch, wobei die Sensorelektronikanordnung mit dem Analytsensor koppelbar ist.

## Revendications

1. Procédé de fabrication d'un lot de systèmes de capteur d'analyte in vivo, comprenant :
la formation de plusieurs systèmes de capteur d'analyte in vivo, chaque système de capteur d'analyte in vivo comprenant :
un capteur d'analyte in vivo (800) comprenant :
un substrat (802) ;
une couche conductrice (804, 804a, 804b) disposée sur au moins une partie du substrat ;
une couche diélectrique (808) disposée sur la couche conductrice et ayant un vide (810) dans celle-ci ;
une couche de détection (806) disposée au sein du vide et formée sur la couche conductrice pour définir une zone active du capteur d'analyte qui génère un ou plusieurs signaux correspondant à un niveau d'analyte surveillé après l'insertion du capteur d'analyte et quand il est maintenu en contact fluidique avec du fluide interstitiel, dans lequel la zone de la couche de détection en contact avec la couche conductrice a un coefficient de variation de capteur-à-capteur inférieur à approximativement 5 % au sein du lot ; et
une couche de membrane formée sur la couche de détection, dans lequel la couche de membrane a une épaisseur définie avec un coefficient de variation capteur-à-capteur inférieur à approximativement 5 % au sein du lot ; et
un ensemble électronique de capteur comprenant un processeur de données,
la détermination de l'épaisseur de la couche de membrane de chaque capteur d'analyte dans le lot et au niveau d'un ou de plusieurs points de la couche de membrane ;
la détermination d'un paramètre d'étalonnage sur la base d'une moyenne de l'épaisseur déterminée des couches de membrane du lot de capteurs d'analyte ; et
le stockage du paramètre d'étalonnage déterminé dans le processeur de données de l'ensemble électronique de capteur de chacun des systèmes de capteur d'analyte in vivo dans le lot, ou la fourniture du paramètre d'étalonnage déterminé avec chacun des systèmes de capteur d'analyte in vivo dans le lot, pendant la fabrication des systèmes de capteur d'analyte ;
dans lequel le processeur de données est configuré pour mettre en corrélation les un ou plusieurs signaux générés avec le niveau d'analyte surveillé en appliquant le paramètre d'étalonnage déterminé aux un ou plusieurs signaux générés.

2. Procédé de fabrication d'un lot de systèmes de capteur d'analyte in vivo selon l'une quelconque des revendications précédentes, comprenant la formation d'un capteur d'analyte in vivo par :
la fourniture du substrat ;
la disposition de la couche conductrice sur au moins une partie du substrat ;
la disposition de la couche diélectrique sur la couche conductrice ;
la formation du vide dans la couche diélectrique ;
la disposition de la couche de détection au sein du vide et sur la couche conductrice pour définir une zone active du capteur d'analyte qui génère un ou plusieurs signaux correspondant à un niveau d'analyte surveillé après l'insertion du capteur d'analyte et quand il est maintenu en contact fluidique avec du fluide interstitiel ; et
la formation de la couche de membrane sur la couche de détection.

3. Procédé de formation d'un capteur d'analyte in vivo selon la revendication 2, dans lequel l'étape de formation d'un vide dans la couche diélectrique comprend la formation du vide avec une plage de dimensions allant d'environ 0,01 mm² à environ 1,0 mm², facultativement d'environ 0,04 mm² à environ 0,36 mm².

4. Procédé de formation d'un capteur d'analyte in vivo selon la revendication 2 ou 3, dans lequel l'étape de formation d'un vide dans la couche diélectrique comprend la formation du vide avec une hauteur qui est progressivement augmentée ou diminuée et/ou ayant une aire de surface profilée de sorte qu'il se termine en pointe, ou varie autrement.

5. Procédé de formation d'un capteur d'analyte in vivo selon la revendication 2, 3 ou 4, dans lequel l'étape de formation d'un vide dans la couche diélectrique comprend la formation du vide en utilisant des techniques de photolithographie, comprenant facultativement une photogravure à une profondeur suffisante pour exposer la couche conductrice.

6. Procédé de fabrication d'un lot de systèmes de capteur d'analyte in vivo selon l'une quelconque des revendications précédentes, comprenant en outre : le chargement du paramètre d'étalonnage déterminé d'au moins l'un des systèmes de capteur d'analyte in vivo dans le lot dans une mémoire associée à ce système de capteur d'analyte.

7. Procédé de formation d'un capteur d'analyte in vivo selon l'une quelconque des revendications 2 à 6, dans lequel la couche diélectrique est formée à partir d'un matériau polymère photosensible ou d'un film photosensible disposé sur la couche conductrice et au moins une partie du substrat.

8. Procédé de formation d'un capteur d'analyte in vivo selon l'une quelconque des revendications 2 à 7, dans lequel la couche de détection est formée avec une aire allant d'environ 0,01 mm² à environ 1,0 mm², facultativement d'environ 0,04 mm² à environ 0,36 mm².

9. Procédé de fabrication d'un lot de systèmes de capteur d'analyte in vivo selon l'une quelconque des revendications précédentes, dans lequel la couche de détection est formée sur la couche conductrice.

10. Procédé de fabrication d'un lot de systèmes de capteur d'analyte in vivo selon l'une quelconque des revendications précédentes, dans lequel l'ensemble électronique de capteur peut être couplé avec le capteur d'analyte.
